# EUROPÄISCHE PATENTSCHRIFT

(11) **EP 2 054 123 B1**
(45) Veröffentlichungstag und Bekanntmachung des Hinweises auf die Patenterteilung: **20.11.2013**
(21) Anmeldenummer: 07857230.2
(22) Anmeldetag: 20.11.2007
(51) Int. Cl.: A61K 8/64, A61K 8/46, A61K 8/49, A61Q 5/06, A61Q 5/08, A61Q 5/10

(54) **HAARFARBVERÄNDERUNG MIT HAUTSCHUTZ**
HAIR COLOR CHANGE WHICH PROTECTS THE SKIN
COLORATION CAPILLAIRE PROTECTRICE POUR LA PEAU

(30) Priorität: 20.12.2006 DE 102006060948
(43) Veröffentlichungstag der Anmeldung: 06.05.2009
(73) Patentinhaber: Henkel AG & Co. KGaA, 40589 Düsseldorf (DE)
(72) Erfinder: DÖRING, Thomas, 41540 Dormagen (DE); BOSSMANN, Britta, 40699 Erkrath (DE)
(86) Internationale Anmeldenummer: PCT/EP2007/062529
(87) Internationale Veröffentlichungsnummer: WO 2008/074579

(56) Entgegenhaltungen:
- WO-A-01/93824
- WO-A-2007/140856
- DE-A- 10 133 197
- DE-A1- 4 419 783
- DE-A1- 10 229 995
- GUALA F. ET AL.: "Enhancement of Wash resistance and Functionality in Hair Colouring by Sodium Methyl Cocoyl Taurate and Sodium Myristoyl Sarcosinate Formulae"[Online] 5. Januar 2006 (2006-01-05), Seiten 1-11, XP002470375 Gefunden im Internet: URL:http://www.erwebhosting.it/zsi/eng_htm l/news.php?id_cate=17> [gefunden am 2008-02-25]

## Beschreibung

Die vorliegende Erfindung betrifft die kosmetische Verwendung einer Kombination aus Aminosulfonsäuren und niedermolekularen Disulfiden während der Haarfarbveränderung zur Verbesserung des Kopfhautempfindens. Ein entsprechendes Verfahren zur farbverändemden Haarbehandlung, sowie die darin verwendbaren Mittel sind ebenso Gegenstand der vorliegenden Erfindung.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten.

Die permanente, oxidative Färbung sowie die Haarbleiche und die Fixierung im Rahmen der Dauerwelle finden meist als oxidative Haarbehandlung in Gegenwart von Oxidationsmitteln, wie Wasserstoffperoxyd, statt. Das Oxidationsmittel erzielt hierbei leider nicht nur die erwünschte kosmetische Wirkung, sondern schädigt zusätzlich die Struktur des Haarkeratins. Das keratineigene Cystin oxidiert dabei zur Cysteinsäure, was sich negativ auf die Stabilität, die Haptik und die Optik der Haarfaser auswirkt. Derart geschädigtes Haar wirkt stumpf und spröde. In extremen Fällen kommt es sogar zum Haarbruch.

Darüber hinaus kommt während der Haarfärbung, insbesondere während der oxidativen Haarfärbung, die Kopfhaut mit dem Mittel in Berührung und der empfindliche Proband spürt während der Haarbehandlung oftmals ein Missempfinden auf der Kopfhaut. Dieses Missempfinden wird meist als Stechen oder Jucken beschrieben.

Gemäß Druckschrift US-A-20030180337 kann α-Liponsäure zur Bekämpfung von oxidativen Schäden des Haars durch Bewitterung eingesetzt werden.

Es ist die Aufgabe der vorliegenden Erfindung, die zuvor beschriebenen Nebenwirkungen farbverändemder Haarbehandlungen bereits während der Färbung zu verringern, ohne den Wirkungsgrad des farbverändemden Kosmetikums, insbesondere bezüglich Farbintensität, Farbechtheit oder Aufhellleistung, zu verschlechtern.

Es wurde nun überraschenderweise gefunden, daß die Aufgabe in hervorragendem Maße durch die Verwendung einer Kombination aus mindestens einer Aminosulfonsäure und mindestens einer niedermolekularen Disulfidverbindung in einem Farbveränderungsmittel gelöst wird. Die Wirkung entfaltet sich während der Haarbehandlung.

Ein erster Gegenstand der Erfindung sind kosmetische Mittel, enthaltend in einem kosmetischen Träger mindestens eine haar farbverändernde Komponente und eine Kombination aus
(i) mindestens einer Verbindung gemäß Formel (I), 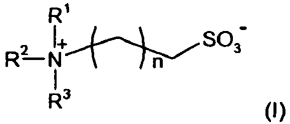 worin
   - R¹, R² und R³: stehen unabhängig voneinander für ein Wasserstoffatom, -CH₃, -CH₂CH₃, -CH(CH₃)₂, -CH₂CH₂CH₃, -CH(CH₃)CH₂CH₃, -CH₂CH(CH₃)₂, -C(CH₃)₃,
   - n: steht für 1 oder 2,
   und
(ii) mindestens einer organischen Disulfidverbindung mit einem Molekulargewicht von kleiner 700 g/mol.

Als kosmetische Träger eignen sich erfindungsgemäß besonders Cremes, Emulsionen, Gele oder auch tensidhaltige schäumende Lösungen, wie beispielsweise Shampoos, Schaumaerosole oder andere Zubereitungen, die insbesondere für die Anwendung auf dem Haar geeignet sind. Es ist aber auch denkbar, die Inhaltsstoffe in eine pulverförmige oder auch tablettenförmige Formulierung zu integrieren, welche vor der Anwendung in Wasser gelöst wird. Die kosmetischen Träger können insbesondere wässrig oder wässrig-alkoholisch sein.

Ein wässriger kosmetischer Träger enthält mindestens 50 Gew.-% Wasser.

Unter wässrig-alkoholischen kosmetischen Trägern sind im Sinne der vorliegenden Erfindung wässrige Lösungen enthaltend 3 bis 70 Gew.-% eines C₁-C₄-Alkohols, insbesondere 1,2-Propylenglykol, Ethanol oder Isopropanol, zu verstehen. Die erfindungsgemäßen Mittel können zusätzlich weitere organische Lösemittel, wie beispielsweise Methoxybutanol, Benzylalkohol oder Ethyldiglykol, enthalten. Bevorzugt sind dabei alle wasserlöslichen organischen Lösemittel.

Als Verbindungen der Formel (I) eignen sich bevorzugt solche, bei denen R¹, R² und R³ für ein Wasserstoffatom oder eine Methylgruppe stehen und n die Zahl 1 oder 2 bedeutet.

Wenn mindestens ein Rest aus R¹, R² oder R³ für ein Wasserstoffatom steht, so liegt die Aminosulfonsäure gemäß Formel (I) mit den Verbindungen der Formel (Ia) bzw. (Ib) im chemischen Gleichgewicht in dem erfindungsgemäßen Mittel vor. Das chemische Gleichgewicht verschiebt sich, je nach pH-Wert des Mittels, in Richtung einer der Verbindungen (I), (Ia) oder (Ib). das Gleichgewicht sei im Folgenden exemplarisch an einer Verbindung der Formel (I) illustriert, in der R³ für ein Wasserstoffatom steht und R¹, R² und n die Bedeutungen gemäß Formel (I) annehmen:

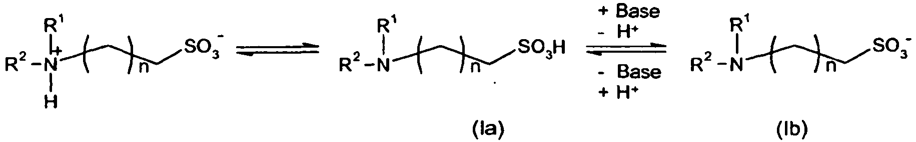

Es ist erfindungsgemäß besonders bevorzugt, wenn als Verbindung der Formel (I) mindestens eine Verbindung aus der Gruppe verwendet wird, die gebildet wird aus 2-Ammonioethansulfonat (Taurin), 2-(N-Methylammonio)ethansulfonat, 2-(N,N-Dimethylammonio)ethansulfonat, 2-(N,N,N-Trimethylammonio)ethansulfonat, 3-Ammoniopropansulfonat (Homotaurin), 3-(N-Methylammonio)propansulfonat, 3-(N,N-Dimethylammonio)propansulfonat, 3-(N,N,N-Trimethylammonio)propansulfonat. Besonders bevorzugt wird 2-Ammonioethansulfonat als Verbindung der Formel (I) in den erfindungsgemäßen Mitteln eingesetzt.

Die erfindungsgemäßen kosmetischen Mittel enthalten die Verbindungen der Formel (I) bevorzugt in einer Menge von 0,01 bis 7,0 Gew.-%, besonders bevorzugt von 0,05 bis 5,0 Gew.-%, ganz besonders bevorzugt von 0,2 bis 2,0 Gew.-%, jeweils bezogen auf das Gewicht des anwendungsbereiten Mittels.

Die erfindungsgemäßen organischen Disulfidverbindungen tragen mindestens einen gegebenenfalls substituierten Kohlenwasserstoffrest. Erfindungsgemäß bevorzugt weisen sie nur eine Disulfidbindung im Molekül auf. Die in dem erfindungsgemäßen Mittel enthaltenen organischen Disulfidverbindungen der Formel (I) besitzen zwingend ein Molekulargewicht von kleiner 700 g/mol, bevorzugt von kleiner 500 g/mol, besonders bevorzugt von kleiner 350 g/mol.

Erfindungsgemäß bevorzugt geeignete organische Disulfidverbindungen werden ausgewählt aus mindestens einer Verbindung der Gruppe, die gebildet wird aus Cystin und α-Liponsäure, sowie den Salzen der vorgenannten Verbindungen.

Die erfindungsgemäßen kosmetischen Mittel enthalten die erfindungsgemäße, organische Disulfidvervindung bevorzugt in einer Menge von 0,01 bis 3,0 Gew.-%, besonders bevorzugt von 0,1 bis 1,0 Gew.-%, jeweils bezogen auf das Gewicht des anwendungsbereiten Mittels.

Als erfindungsgemäß bevorzugtes organisches Disulfid enthalten die erfindungsgemäßen Mittel bevorzugt α-Liponsäure.

Die α-Liponsäure kann erfindungsgemäß als freie Säure, aber auch in Gestalt der jeweiligen Salze eingesetzt werden. Alle weiteren Ausführungen erstrecken sich, selbst wenn lediglich α-Liponsäure erwähnt wird, selbstredend sowohl auf die freie Säure und auf die Salzform. Wenn die α-Liponsäure in den erfindungsgemäßen Mitteln teilweise reduziert wird, enthalten die erfindungsgemäßen Mittel zwangsläufig Dihydrollponsäure.

Die erfindungsgemäß verwendete α-Liponsäure bzw. die Dihydroliponsäure gehorchen somit der Formel (ii-1) bzw. Formel (ii-2), 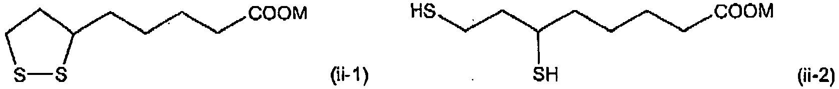 worin jeweils M ein Wasserstoffatom oder ein Äquivalent eines ein- oder mehrwertigen Kations bedeutet.

Wenn die Verbindungen der Formeln (ii-1) bzw. (ii-2) als Säure vorliegen, bedeutet der Rest M ein Wasserstoffatom. Das Fragment -COOM der Formel (ii-1) bzw. (ii-2) bildet in diesem Fall eine Carboxylgruppe. Wenn die Verbindungen der Formeln (ii-1) bzw. (ii-2) als Salz vorliegen, steht M für ein Äquivalent eines ein- oder mehrwertigen Kations. Das ein oder mehrwertige Kation M^{z+} mit einer Ladungszahl z von eins oder höher dient lediglich aus Gründen der Elektroneutralität zur Kompensation der einfach negativen Ladung des bei Salzbildung vorliegenden Carboxylatfragments -COO⁻ in Formel (ii-1) bzw. (ii-2). Das dafür zu verwendende Äquivalent des entsprechenden Kations beträgt 1/z. Das Fragment -COOM der Formeln (ii-1) bzw. (ii-2) steht im Fall der Salzbildung für die Gruppe: -COO⁻ 1/z (M^{z+}).

Als ein- oder mehrwertige Kationen M^{z+} kommen prinzipiell alle physiologisch verträglichen Kationen in Frage. Insbesondere sind dies Metallkationen der physiologisch verträglichen Metalle aus den Gruppen la, Ib, IIa, IIb, IIIb, VIa oder VIII des Periodensystems der Elemente, Ammoniumionen, sowie kationische organische Verbindungen mit quaterniertem Stickstoffatom. Letztere werden beispielsweise durch Protonierung primärer, sekundärer oder tertiärer organischer Amine mit einer Säure, wie z.B. mit Verbindungen der Formel (ii-1) in ihrer sauren Form, oder durch permanente Quaternisierung besagter organischer Amine gebildet. Beispiele dieser kationischen organischen Ammoniumverbindungen sind 2-Ammonioethanol und 2-Trimethylammonioethanol. M steht in Formel (ii-1) bzw. (ii-2) bevorzugt für ein Wasserstoffatom, ein Ammoniumion, ein Alkalimetallion, für ein halbes Äquivalent eines Erdalkalimetallions oder ein halbes Äquivalent eines Zinkions, besonders bevorzugt für ein Wasserstoffatom, ein Ammoniumion, ein Natriumion, ein Kaliumion, ½ Kalziumion, ½ Magnesiumion oder ½ Zinkion.

Die erfindungsgemäß verwendete α-Liponsäure trägt ein chirales Zentrum am Kohlenstoffatom der sechsten Position des Kohlenstoffgerüstes. Für die erfindungsgemäße Verwendung ist die Stereochemie der α-Liponsäure unwesentlich. Folglich können die enantiomerenreinen Formen als auch beliebige Mischungen der Enantiomeren (wie insbesondere Racemate) erfindungsgemäß verwendet werden.

Es ist erfindungsgemäß bevorzugt die Verbindungen der Formel (I) und die organischen Disulfide mit einem Molekulargewicht kleiner 700 g/mol in einem Gewichtsverhältnisbereich (Formel (I) zu Disulfid) von 10 zu 1 bis 1 zu 1, besonders bevorzugt von 5 zu 1 bis 2 zu 1, einzusetzen.

Es ist ganz besonders bevorzugt, wenn das erfindungsgemäße Mittel als Verbindung der Formel (I) 2-Ammonioethansulfonat und als organisches Disulfid mit einem Molekulargewicht kleiner 700 g/mol α-Liponsäure gemäß Formel (ii-1) enthält. Dabei sind wiederum die oben angegebenen Mengenangaben und Mengenverhältnisse der beiden Komponenten bevorzugt.

Die erfindungsgemäßen Mittel besitzen bevorzugt einen pH-Wert im Bereich von 6 bis 12, insbesondere von pH 7,5 bis 10, ganz besonders bevorzugt von pH 8 bis 10.

Die kosmetischen Mittel enthalten zwingend mindestens eine haar farbverändernde Komponente.

Die haar farbverändernde Komponente wird bevorzugt ausgewählt
(a) aus mindestens einem Oxidationsfarbstoffvorprodukt vom Typ der Entwicklerkomponenten und gegebenenfalls zusätzlich mindestens einer Kupplerkomponente
   und/oder
(b) aus Oxofarbstoffvorprodukten
   und/oder
(c) aus mindestens einem direktziehenden Farbstoff
   und/oder
(d) aus mindestens einer Vorstufe naturanaloger Farbstoffe
   und/oder
(e) aus mindestens einem Oxidationsmittel und gegebenenfalls zusätzlich mindestens einem Bleichverstärker.

Als Entwickterkomponenten werden üblicherweise primäre aromatische Amine mit einer weiteren, in para- oder ortho-Position befindlichen, freien oder substituierten Hydroxy- oder Aminogruppe, heterozyklische Hydrazone, 4-Aminopyrazolderivate sowie 2,4,8,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 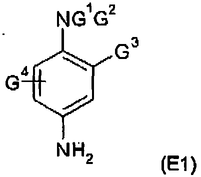 wobei
- G¹ steht für ein Wasserstoffatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest, einen 4'-Aminophenylrest oder einen (C₁ bis C₄)-Alkylrest, der mit einer stickstoffhaltigen Gruppe, einem Phenyl- oder einem 4'-Aminophenylrest substituiert ist;
- G² steht für ein Wasserstoffatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest oder einen (C₁ bis C₄)-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Hydroxyalkoxyrest, einen (C₁ bis C₄)-Acetylaminoalkoxyrest, einen Mesylamino-(C₁ bis C₄)-alkoxyrest oder einen (C₁ bis C₄)-Carbamoylaminoalkoxyrest;
- G⁴ steht für ein Wasserstoffatom, ein Halogenatom oder einen (C₁ bis C₄)-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Besonders bevorzugte p-Phenylendiamine der Formel (E1) werden ausgewählt aus einer oder mehrerer Verbindungen der Gruppe, die gebildet wird, aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin, 5,8-Diaminobenzo-1,4-dioxan sowie ihren physiologisch verträglichen Salzen.

Erfindungsgemäß ganz besonders bevorzugte p-Phenylendiaminderivate der Formel (E1) sind ausgewählt aus mindestens einer Verbindung der Gruppe p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 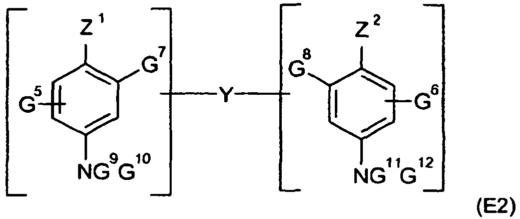 wobei:
- Z¹ und Z² stehen unabhängig voneinander für einen Hydroxyl- oder NH₂-Rest, der gegebenenfalls durch einen (C₁ bis C₄)-Alkylrest, durch einen (C₁ bis C₄)-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder (C₁ bis C₈)-Alkoxyreste substituiert sein kann, oder eine direkte Bindung,
- G⁵ und G⁶ stehen unabhängig voneinander für ein Wasserstoff- oder Halogenatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen (C₁ bis C₄)-Alkylrest,
mit der Maßgabe, dass die Verbindungen der Formel (E2) nur eine Verbrückung Y pro Molekül enthalten.

Die in Formel (E2) verwendeten Substituenten sind erfindungsgemäß analog zu den obigen Ausführungen definiert.

Bevorzugte zweikernige Entwicklerkomponenten der Formel (E2) werden insbesondere aus mindestens einer der folgenden Verbindungen ausgewählt: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4'-aminophenyl)-tetramethylendiamin, N,N'-Bis-(β-hydroxyethyl)-N,N'-bis-(4'-aminophenyl)-tetramethylendiamin, N,N'-Bis-(4-(methylamino)phenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan sowie ihre physiologisch verträglichen Salze.

Ganz besonders bevorzugte zweikernige Entwicklerkomponenten der Formel (E2) werden ausgewählt unter N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadocan oder eines der physiologisch verträglichen Salze dieser Verbindungen.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 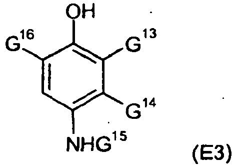 wobei:
- G¹³ steht für ein Wasserstoffatom, ein Halogenatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest, einen (C₁ bis C₄)-Aminoalkylrest, einen Hydroxy-(C₁ bis C₄)-alkylaminorest, einen (C₁ bis C₄)-Hydroxyalkoxyrest, einen (C₁ bis C₄)-Hydroxyalkyl-(C₁ bis C₄)-aminoalkylrest oder einen (Di-[(C₁ bis C₄)-alkyl]amino)-(C₁ bis C₄)-alkylrest, und
- G¹⁴ steht für ein Wasserstoff- oder Halogenatom, einen (C₁ bis C₄)-Alkylrest, einen (C₁ bis C₄)-Monohydroxyalkylrest, einen (C₂ bis C₄)-Polyhydroxyalkylrest, einen (C₁ bis C₄)-Alkoxy-(C₁ bis C₄)-alkylrest, einen (C₁ bis C₄)-Aminoalkylrest oder einen (C₁ bis C₄)-Cyanoalkylrest,
- G¹⁵ steht für Wasserstoff, einen (C₁ bis C₄)-Alkylrest, einen (C₁ bis C₄)-Monohydroxyalkylrest, einen (C₂ bis C₄)-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidinderivaten, Pyrazolderivaten, Pyrazolopyrimidin-Derivaten bzw. ihren physiologisch verträglichen Salzen.

Bevorzugte Pyrimidin-Derivate werden erfindungsgemäß ausgewählt aus Verbindungen gemäß Formel (E4) bzw. deren physiologisch verträglichen Salzen, 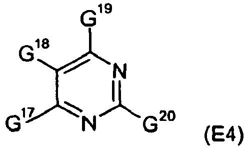 worin
- G¹⁷, G¹⁸ und G¹⁹ unabhängig voneinander für ein Wasserstoffatom, eine Hydroxygruppe, eine (C₁ bis C₄)-Alkoxygruppe oder eine Aminogruppe steht und
- G²⁰ für eine Hydroxygruppe oder eine Gruppe -NG²¹G²² steht, worin G²¹ und G²² unabhängig voneinander stehen für ein Wasserstoffatom, eine (C₁ bis C₄)-Alkylgruppe, eine (C₁ bis C₄)-Monohydroxyalkylgruppe,
mit der Maßgabe, dass maximal zwei der Gruppen aus G¹⁷, G¹⁸, G¹⁹ und G²⁰ eine Hydroxygruppe bedeuten und höchstens zwei der Reste G¹⁷, G¹⁸ und G¹⁹ für ein Wasserstoffatom stehen. Dabei ist es wiederum bevorzugt, wenn gemäß Formel (E4) mindestens zwei Gruppen aus G¹⁷, G¹⁸, G¹⁹ und G²⁰ für eine Gruppe -NG²¹G²² stehen und höchstens zwei Gruppen aus G¹⁷, G¹⁸, G¹⁹ und G²⁰ für eine Hydroxygruppe stehen.

Besonders bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate werden erfindungsgemäß ausgewählt aus Verbindungen gemäß Formel (E5), 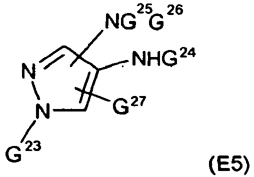 worin
- G²³, G²⁴, G²⁵ stehen unabhängig voneinander für ein Wasserstoffatom, eine (C₁ bis C₄)-Alkylgruppe, eine (C₁ bis C₄)-Monohydroxyalkylgruppe, eine (C₂ bis C₄)-Polyhydroxyalkylgruppe, eine gegebenenfalls substituierte Arylgruppe oder eine gegebenenfalls substituierte Aryl-(C₁ bis C₄)-alkylgruppe, mit der Maßgabe dass, wenn G²⁵ für ein Wasserstoffatom steht, G²⁶ neben den vorgenannten Gruppen zusätzlich für eine Gruppe - NH₂ stehen kann,
- G²⁶ steht für ein Wasserstoffatom, eine (C₁ bis C₄)-Alkylgruppe, eine (C₁ bis C₄)-Monohydroxyalkylgruppe oder eine (C₂ bis C₄)-Polyhydroxyalkylgruppe und
- G²⁷ steht für ein Wasserstoffatom, eine gegebenenfalls substituierte Arylgruppe, eine (C₁ bis C₄)-Alkylgruppe oder eine (C₁ bis C₄)-Monohydroxyalkylgruppe, insbesondere für ein Wasserstoffatom oder eine Methylgruppe.

Bevorzugt bindet in Formel (E5) der Rest -NG²⁵G²⁶ an die 5 Position und der Rest G²⁷ an die 3 Position des Pyrazolzyklus.

Besonders bevorzugte Pyrazol-Derivate sind insbesondere die Verbindungen, die ausgewählt werden unter 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methyl-pyrazol, 4,5-Diamino-3-hydroxymethyl-1-isopropylpyrazol, 4,5-Diamino-3-methyl-1-isopropyl-pyrazol, 4-Amino-5-(β-aminoethyl)amino-1,3-dimethylpyrazol, sowie deren physiologisch verträglichen Salze.

Bevorzugte Pyrazolopyrimidin-Derivate sind insbesondere die Derivate des Pyrazolo[1,5-a]pyrimidin der folgenden Formel (E6) und dessen tautomeren Formen, sofern ein tautomeres Gleichgewicht besteht: 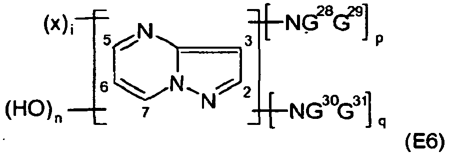 wobei:
- G²⁸, G²⁹ und G³⁰, G³¹ unabhängig voneinander stehen für ein Wasserstoffatom, einen (C₁ bis C₄)-Alkylrest, einen Aryl-Rest, einen (C₁ bis C₄)-Monohydroxyalkylrest, einen (C₂ bis C₄)-Polyhydroxyalkylrest einen (C₁ bis C₄)-Alkoxy-(C₁ bis C₄)-alkylrest, einen (C₁ bis C₄)-Aminoalkylrest, der gegebenenfalls durch ein Acetyl-Ureid- oder einen Sulfonyl-Rest geschützt sein kann, einen (C₁ bis C₄)-Alkylamino-(C₁ bis C₄)-alkylrest, einen Di-[(C₁ bis C₄)-alkyl]-(C₁ bis C₄)-aminoalkylrest, wobei die Dialkyl-Reste gegebenenfalls einen Kohlenstoffzyklus oder einen Heterozyklus mit 5 oder 6 Kettengliedern bilden, einen (C₁ bis C₄)-Monohydroxyalkyl- oder einen Di[(C₁ bis C₄)-Hydroxyalkyl]-(C₁ bis C₄)-aminoalkylrest,
- die X-Reste stehen unabhängig voneinander für ein Wasserstoffatom, einen (C₁ bis C₄)-Alkylrest, einen Aryl-Rest, einen (C₁ bis C₄)-Monohydroxyalkylrest, einen (C₂ bis C₄)-Polyhydroxyalkylrest, einen (C₁ bis C₄)-Aminoalkylrest, einen (C₁ bis C₄)-Alkylamino-(C₁ bis C₄)-alkylrest, einen Di-[(C₁ bis C₄)alkyl]-(C₁ bis C₄)-aminoalkylrest, wobei die Dialkyl-Reste gegebenenfalls einen Kohlenstoffzyklus oder einen Heterozyklus mit 5 oder 6 Kettengliedern bilden, einen (C₁ bis C₄)-Hydroxyalkyl- oder einen Di-[(C₁ bis C₄)-hydroxyalkyl]amino-(C₁ bis C₄)-alkylrest, einen Aminorest, einen (C₁ bis C₄)-Alkyl- oder Di-[(C₁ bis C₄)-hydroxyalkyl]aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
   mit der Maßgabe, dass
- die Summe aus p + q ungleich 0 ist,
- wenn p + q gleich 2 ist, n den Wert 0 hat, und die Gruppen NG²⁸G²⁹ und NG³⁰G³¹ belegen die Positionen (2,3); (5,6); (6,7); (3,5) oder (3,7);
- wenn p + q gleich 1 ist, n den Wert 1 hat, und die Gruppen NG²⁸G²⁹ (oder NG³⁰G³¹) und die Gruppe OH belegen die Positionen (2,3); (5,6); (6,7); (3,5) oder (3,7);

Die in Formel (E7) verwendeten Substituenten sind erfindungsgemäß analog zu den obigen Ausführungen definiert.

Wenn das Pyrazolo[1,5-a]pyrimidin der obenstehenden Formel (E6) eine Hydroxygruppe an einer der Positionen 2, 5 oder 7 des Ringsystems enthält, besteht ein tautomeres Gleichgewicht, das zum Beispiel im folgenden Schema dargestellt wird:

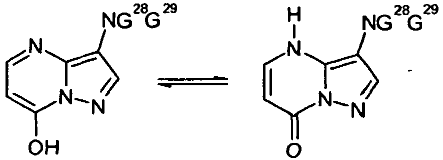

Unter den Pyrazolo[1,5-a]pyrimidinen der obenstehenden Formel (E7) kann man insbesondere nennen:
- Pyrazolo[1,5-a]pyrimidin-3,7-diamin;
- 2,5-Dimethyl-pyrazolo[1,5-a]pyrimidin-3,7-diamin;
- Pyrazolo[1,5-a]pyrimidin-3,5-diamin;
- 2,7-Dimethyl-pyrazolo[1,5-a]pyrimidin-3,5-diamin;
- 3-Aminopyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 2-(7-Aminopyrazolo[1,5-a]pyrimidin-3-ylamino)-ethanol;
- 2-[(3-Aminopyrazolo[1,5-a]pyrimidin-7-yl)-(2-hydroxy-ethyl)-amino]-ethanol;
- 2-[(7-Aminopyrazolo[1,5-a]pyrimidin-3-yl)-(2-hydroxy-ethyl)-amino]-ethanol;
- 5,6-Dimethylpyrazolo[1,5-a]pyrimidin-3,7-diamin;
- 2,6-Dimethylpyrazolo[1,5-a]pyrimidin-3,7-diamin;
- 3-Amino-7-dimethylamino-2,5-dimethylpyrazolo[1,5-a]pyrimidin;
sowie ihre physiologisch verträglichen Salze und ihre tautomeren Formen, wenn ein tautomers Gleichgewicht vorhanden ist.

Die Pyrazolo[1,5-a]pyrimidine der obenstehenden Formel (E6) können wie in der Literatur beschrieben durch Zyklisierung ausgehend von einem Aminopyrazol oder von Hydrazin hergestellt werden.

Ganz besonders bevorzugte Entwicklerkomponenten werden ausgewählt, aus mindestens einer Verbindung aus der Gruppe, die gebildet wird aus p-Phenylendiamin, p-Toluylendiamin, 2-(β-Hydroxyethyl)-p-phenylendiamin, 2-(α,β-Dihydroxyethyl)-p-phenylendiamin, N,N-Bis-(β-hydroxyethyl)-p-phenylendiamin, N-(4-Amino-3-methylphenyl)-N-[3-(1H-imidazol-1-yl)propyl]amin, N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(α,β-dihydroxyethyl)-phenol und 4-Amino-2-(diethylaminomethyl)-phenol, 4,5-Diamino-1-(β-hydroxyethyl)-pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen.

Im folgenden werden Beispiele für die als Substituenten der Verbindungen der Formeln (E1) bis (E6) genannten Reste aufgezählt: Beispiele für (C₁ bis C₄)-Alkylreste sind die Gruppen -CH₃, - CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃. Erfindungsgemäße Beispiele für (C₁ bis C₄)-Alkoxyreste sind -OCH₃, -OCH₂CH₃, -OCH₂CH₂CH₃, -OCH(CH₃)₂, -OCH₂CH₂CH₂CH₃, -OCH₂CH(CH₃)₂, -OCH(CH₃)CH₂CH₃, - OC(CH₃)₃, insbesondere eine Methoxy- oder eine Ethoxygruppe. Weiterhin können als bevorzugte Beispiele für eine (C₁ bis C₄)-Monohydroxyalkylgruppe -CH₂OH,
-CH₂CH₂OH, -CH₂CH₂CH₂OH, -CHCH(OH)CH₃, -CH₂CH₂CH₂CH₂OH, wobei die Gruppe - -CH₂CH₂OH bevorzugt ist.
Ein besonders bevorzugtes Beispiel einer (C₂ bis C₄)-Polyhydroxyalkylgruppe ist die 1,2-Dihydroxyethylgruppe.
Beispiele für Halogenatome sind F-, Cl- oder Br-Atome, Cl-Atome sind ganz besonders bevorzugte Beispiele.
Beispiele für stickstoffhaltige Gruppen sind insbesondere -NH₂, (C₁ bis C₄)-Monoalkylaminogruppen, (C₁ bis C₄)-Dialkylaminogruppen, (C₁ bis C₄)-Trialkylammoniumgruppen, (C₁ bis C₄)-Monohydroxyalkylaminogruppen, Imidazolinium und - NH₃⁺.
Beispiele für (C₁ bis C₄)-Monoalkylaminogruppen sind -NHCH₃, -NHCH₂CH₃, -NHCH₂CH₂CH₃, -NHCH(CH₃)₂.
Beispiele für (C₁ bis C₄)-Dialkylaminogruppe sind -N(CH₃)₂, -N(CH₂CH₃)₂.
Beispiele für (C₁ bis C₄)-Trialkylammoniumgruppen sind -N⁺(CH₃)₃, -N⁺(CH₃)₂(CH₂CH₃), -N⁺(CH₃)(CH₂CH₃)₂.
Beispiele für (C₁ bis C₄)-Hydroxyalkylaminoreste sind -NH-CH₂CH₂OH, -NH-CH₂CH₂OH, -NH-CH₂CH₂CH₂OH, -NH-CH₂CH₂CH₂OH.
Beispiele für (C₁ bis C₄)-Alkoxy-(C₁ bis C₄)-alkylgruppen sind die Gruppen -CH₂CH₂-O-CH₃, -CH₂CH₂CH₂-O-CH₃, -CH₂CH₂-O-CH₂CH₃, -CH₂CH₂CH₂-O-CH₂CH₃, -CH₂CH₂-O-CH(CH₃), -CH₂CH₂CH₂-O-CH(CH₃).
Beispiele für Hydroxy-(C₁ bis C₄)-alkoxyreste sind -O-CH₂OH, -O-CH₂CH₂OH, -O-CH₂CH₂CH₂OH, -O-CHCH(OH)CH₃, -O-CH₂CH₂CH₂CH₂OH.
Beispiele für (C₁ bis C₄)-Acetylaminoalkoxyreste sind -O-CH₂NHC(O)CH₃, -O-CH₂CH₂NHC(O)CH₃, -O-CH₂CH₂CH₂NHC(O)CH₃, -O-CH₂CH(NHC(O)CH₃)CH₃, -O-CH₂CH₂CH₂CH₂NHC(O)CH₃.
Beispiele für (C₁ bis C₄)-Carbamoylaminoalkoxyreste sind -O-CH₂CH₂-NH-C(O)-NH₂, -O-CH₂CH₂CH₂-NH-C(O)-NH₂, -O-CH₂CH₂CH₂CH₂-NH-C(O)-NH₂.
Beispiele für (C₁ bis C₄)-Aminoalkylreste sind -CH₂NH₂, -CH₂CH₂NH₂, -CH₂CH₂CH₂NH₂, -CH₂CH(NH₂)CH₃, -CH₂CH₂CH₂CH₂NH₂.
Beispiele für (C₁ bis C₄)-Cyanoalkylreste sind -CH₂CN, -CH₂CH₂CN, -CH₂CH₂CH₂CN.
Beispiele für (C₁ bis C₄)-Hydroxyalkylamino-(C₁ bis C₄)-alkylreste sind -CH₂CH₂NH-CH₂CH₂OH, -CH₂CH₂CH₂NH-CH₂CH₂OH, -CH₂CH₂NH-CH₂CH₂CH₂OH, -CH₂CH₂CH₂NH-CH₂CH₂CH₂OH.
Beispiele für Di[(C₁ bis C₄)-Hydroxyalkyl]amino-(C₁ bis C₄)-alkylreste sind - CH₂CH₂N(CH₂CH₂OH)₂, -CH₂CH₂CH₂N(CH₂CH₂OH)₂, -CH₂CH₂N(CH₂CH₂CH₂OH)₂, -CH₂CH₂CH₂N(CH₂CH₂CH₂OH)₂.
Ein Beispiel für Arylgruppen ist die Phenylgruppe.
Beispiele für Aryl-(C₁ bis C₄)-alkylgruppen sind die Benzylgruppe und die 2-Phenylethylgruppe.

Als Kupplerkomponenten werden in der Regel m-Phenylendiaminderivate, Naphthole, Resorcin und Resorcinderivate, Pyrazolone und m-Aminophenolderivate sowie heterozyklische Verbindungen verwendet.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol
sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin und die physiologisch verträglichen Salzen der vorgenannten Verbindungen.

Die erfindungsgemäßen, kosmetischen Mittel enthalten die Entwicklerkomponenten bevorzugt in einer Menge von 0,005 bis 10 Gew.-%, vorzugsweise von 0,1 bis 5 Gew.-%, jeweils bezogen auf das gesamte Mittel.

Die erfindungsgemäßen, kosmetischen Mittel enthalten die Kupplerkomponenten bevorzugt in einer Menge von 0,005 bis 10 Gew.-%, vorzugsweise von 0,1 bis 5 Gew.-%, jeweils bezogen auf das gesamte Mittel.

Das erfindungsgemäße Mittel kann als farbverändernde Komponente in Form der Oxofarbstoffvorprodukte mindestens eine Kombination, aus mindestens einer Verbindung der Komponente
1 Verbindungen, die eine reaktive Carbonylgruppe enthalten mit mindestens einer Verbindung der Komponente
2 Verbindungen, ausgewählt aus (a) CH-aciden Verbindungen, (b) Verbindungen mit primärer oder sekundärer Aminogruppe oder Hydroxygruppe, ausgewählt aus primären oder sekundären aromatischen Aminen, stickstoffhaltigen heterozyklischen Verbindungen und aromatischen Hydroxyverbindungen
enthalten.

Erfindungsgemäße Verbindungen mit einer reaktiven Carbonylgruppe (im Folgenden auch reaktive Carbonylverbindungen oder Komponente 1 genannt) besitzen mindestens eine Carbonylgruppe als reaktive Gruppe, welche mit den Verbindungen der Komponente 2 unter Ausbildung einer beide Komponenten verknüpfenden chemischen Bindung reagiert. Ferner sind erfindungsgemäß auch solche Verbindungen als Komponente 1 umfaßt, in denen die reaktive Carbonylgruppe derart derivatisiert bzw. maskiert ist, daß die Reaktivität des Kohlenstoffatoms der derivatisierten bzw. maskierten Carbonylgruppe gegenüber der Komponente 2 stets vorhanden ist. Diese Derivate sind bevorzugt Kondensationsverbindungen von reaktiven Carbonylverbindungen mit
a) Aminen und deren Derivate unter Bildung von Iminen oder Oximen als Kondensationsverbindung
b) Alkoholen unter Bildung von Acetalen oder Ketalen als Kondensationsverbindung
c) Wasser unter Bildung von Hydraten als Kondensationsverbindung von Aldehyden.

Die Komponente 1 wird bevorzugt ausgewählt aus der Gruppe, die gebildet wird aus Acetophenon, Propiophenon, 2-Hydroxyacetophenon, 3-Hydroxyacetophenon, 4-Hydroxyacetophenon, 2-Hydroxypropiophenon, 3-Hydroxypropiophenon, 4-Hydroxypropiophenon, 2-Hydroxybutyrophenon, 3-Hydroxybutyrophenon, 4-Hydroxybutyrophenon, 2,4-Dihydroxyacetophenon, 2,5-Dihydroxyacetophenon, 2,6-Dihydroxyacetophenon, 2,3,4-Trihydroxyacetophenon, 3,4,5-Trihydroxyacetophenon, 2,4,6-Trihydroxyacetophenon, 2,4,6-Trimethoxyacetophenon, 3,4,5-Trimethoxyacetophenon, 3,4,5-Trimethoxy-acetophenon-diethylketal, 4-Hydroxy-3-methoxy-acetophenon, 3,5-Dimethoxy-4-hydroxyacetophenon, 4-Aminoacetophenon, 4-Dimethylaminoacetophenon, 4-Morpholinoacetophenon, 4-Piperidinoacetophenon, 4-Imidazolinoacetophenon, 2-Hydroxy-5-brom-acetophenon, 4-Hydroxy-3-nitroacetophenon, Acetophenon-2-carbonsäure, Acetophenon-4-carbonsäure, Benzophenon, 4-Hydroxybenzophenon, 2-Aminobenzophenon, 4,4'-Dihydroxybenzophenon, 2,4-Dihydroxybenzophenon, 2,4,4'-Trihydroxybenzophenon, 2,3,4-Trihydroxybenzophenon, 2-Hydroxy-1-acetonaphthon, 1-Hydroxy-2-acetonaphthon, Chromon, Chromon-2-carbonsäure, Flavon, 3-Hydroxyflavon, 3,5,7-Trihydroxyflavon, 4',5,7-Trihydroxyflavon, 5,6,7-Trihydroxyflavon, Quercetin, 1-Indanon, 9-Fluorenon, 3-Hydroxyfluorenon, Anthron, 1,8-Dihydroxyanthron, Vanillin, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxybenzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-3-methyl-benzaldehyd, 4-Hydroxy-2,3-dimethylbenzaldehyd, 4-Hydroxy-2,5-dimethyl-benzaldehyd, 4-Hydroxy-2,6-dimethyl-benzaldehyd, 4-Hydroxy-3,5-dimethoxy-benzaldehyd, 4-Hydroxy-3,5-dimethyl-benzaldehyd, 3,5-Diethoxy-4-hydroxy-benzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,5-Dihydroxybenzaldehyd, 2,6-Dihydroxybenzaldehyd, 3,4-Dihydroxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Hydroxy-2-methoxybenzaldehyd, 4-Dimethylaminobenzaldehyd, 4-Diethylaminobenzaldehyd, 4-Dimethylamino-2-hydroxybenzaldehyd, 4-Diethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaldehyd, 4-Hydroxy-1-naphthaldehyd, 4-Dimethylamino-1-naphthaldehyd, 2-Methoxyzimtaldehyd, 4-Methoxy-zimtaldehyd, 4-Hydroxy-3-methoxy-zimtaldehyd, 3,5-Dimethoxy-4-hydroxy-zimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-Dimethylamino-2-methoxybenzaldehyd, 4-(1-Imidazolyl)-benzaldehyd, Piperonal, 2,3,6,7-Tetrahydro-1H,5H-benzo[ij]chinolizin-9-carboxaldehyd, 2,3,6,7-Tetrahydro-8-hydroxy-1H,5H-benzo[ij]chinolizin-9-carboxaldehyd, N-Ethylcarbazol-3-aldehyd, 2-Formylmethylen-1,3,3-trimethylindolin (Fischers Aldehyd oder Tribasen Aldehyd),
2-Indolaldehyd, 3-Indolaldehyd, 1-Methylindol-3-aldehyd, 2-Methylindol-3-aldehyd, 1-Acetylindol-3-aldehyd, 3-Acetylindol, 1-Methyl-3-acetylindol, 2-(1',3',3'-Trimethyl-'2-indolinyliden)-acetaldehyd, 1-Methylpyrrol-2-aldehyd, 1-Methyl-2-acetylpyrrol, 4-Pyridinaldehyd, 2-Pyridinaldehyd, 3-Pyridinaldehyd, 4-Acetylpyridin, 2-Acetylpyridin, 3-Acetylpyridin, Pyridoxal, Chinolin-3-aldehyd, Chinolin-4-aldehyd, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'- Furyl)-acrolein und Imidazol-2-aldehyd, 1,3-Diacetylbenzol, 1,4-Diacetylbenzol, 1,3,5-Triacetylbenzol, 2-Benzoyl-acetophenon, 2-(4'-Methoxybenzoyl)-acetophenon, 2-(2'-Furoyl)-acetophenon, 2-(2'-Pyridoyl)-acetophenon und 2-(3'-Pyridoyl)-acetophenon,
Benzylidenaceton, 4-Hydroxybenzylidenaceton, 2-Hydroxybenzylidenaceton, 4-Methoxybenzylidenaceton, 4-Hydroxy-3-methoxybenzylidenaceton, 4-Dimethylaminobenzylidenaceton, 3,4-Methylendioxybenzylidenaceton, 4-Pyrrolidinobenzylidenaceton, 4-Piperidinobenzylidenaceton, 4-Morpholinobenzylidenaceton, 4-Diethylaminobenzylidenaceton, 3-Benzyliden-2,4-pentandion, 3-(4'-Hydroxybenzyliden)-2,4-pentandion, 3-(4'-Dimethylaminobenzyliden)-2,4-pentandion, 2-Benzylidencyclohexanon, 2-(4'-Hydroxybenzyliden)-cyclohexanon, 2-(4'-Dimethylaminobenzyliden)-cyclohexanon, 2-Benzyliden-1,3-cyclohexandion, 2-(4'-Hydroxybenzyliden)-1,3-cyclohexandion, 3-(4'-Dimethylaminobenzyliden)-1,3-cyclohexandion, 2-Benzyliden-5,5-dimethyl-1,3-cyclohexandion, 2-(4'-Hydroxybenzyliden)-5,5-dimethyl-1,3-cyclohexandion, 2-(4'-Hydroxy-3-methoxybenzyliden)-5,5-dimethyl-1,3-cyclohexandion, 2-(4'-Dimethylaminobenzyliden)-5,5-dimethyl-1,3-cyclohexandion, 2-Benzylidencyclopentanon, 2'-(4-Hydroxybenzyliden)-cyclopentanon, 2-(4'-Dimethylaminobenzyliden)-cyclopentanon, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 6-(4-Dimethylaminophenyl)hexa-3,5-dien-2-on, 6-(4-Diethylaminophenyl)hexa-3,5-dien-2-on, 6-(4-Methoxyphenyl)hexa-3,5-dien-2-on, 6-(3,4-Dimethoxyphenyl)hexa-3,5-dien-2-on, 6-(2,4-Dimethoxyphenyl)hexa-3,5-dien-2-on, 6-(4-Piperidinophenyl)hexa-3,5-dien-2-on, 6-(4-Morpholinophenyl)hexa-3,5-dien-2-on, 6-(4-Pyrrolidinophenyl)hexa-3,5-dien-2-on, 5-(4-Dimethylamino-1-naphthyl)penta-3,5-dienal, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 4-Hydroxy-3-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Nitro-1-naphthaldehyd, 2-Nitrozimtaldehyd, 3-Nitrozimtaldehyd, 4-Nitrozimtaldehyd, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd, 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 4-Acetyl-1-methylpyridinium-, 2-Acetyl-1-methylpyridinium-, 4-Acetyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-, 7-Formyl-1-benzylchinolinium-, 8-Formyl-1-benrylchinolinium, 5-Formyl-1-allylchinolinium-, 6-Formyl-1-allylchinolinium-, 7-Formyl-1-allylchinolinium- und 8-Formyl-1-allylchinolinium-, 5-Acetyl-1-methylchinolinium-, 6-Acetyl-1-methylchinolinium-, 7-Acetyl-1-methylchinolinium-, 8-Acetyl-1-methylchinolinium, 5-Acetyl-1-ethylchinolinium-, 6-Acetyl-1-ethylchinolinium-, 7-Acetyl-1-ethylchinolinium-, 8-Acetyl-1-ethylchinolinium, 5-Acetyl-1-benzylchinolinium-, 6-Acetyl-1-benzylchinolinium-, 7-Acetyl-1-benzylchinolinium-, 8-Acetyl-1-benzylchinolinium, 5-Acetyl-1-allylchinolinium-, 6-Acetyl-1-allylchinolinium-, 7-Acetyl-1-allylchinolinium- und 8-Acetyl-1-allylchinolinium, 9-Formyl-10-methylacridinium-, 4-(2'-Formylvinyl)-1-methylpyridinium-, 1,3-Dimethyl-2-(4'-formylphenyl)-benzimidazolium-, 1,3-Dimethyl-2-(4'-formylphenyl)-imidazolium-, 2-(4'-Formylphenyl)-3-methylbenzothiazolium-, 2-(4'-Acetylphenyl)-3-methylbenzothiazolium-, 2-(4'-Formylphenyl)-3-methylbenzoxazolium-, 2-(5'-Formyl-2'-furyl)-3-methylbenzothiazolium-, 2-(5'-Formyl-2'-furyl)-3-methylbenzothiazolium-, 2-(5'-Formyl-2'-thienyl)-3-methylbenzothiazolium-, 2-(3'-Formylphenyl)-3-methylbenzothiazolium-, 2-(4'-Formyl-1-naphthyl)-3-methylbenzothiazolium-, 5-Chlor-2-(4'-formylphenyl)-3-methylbenzothiazolium-, 2-(4'-Formylphenyl)-3,5-dimethylbenzothiazolium-Salze, bevorzugt mit Benzolsulfonat, p-Toluolsulfonat, Methansulfonat, Perchlorat, Sulfat, Chlorid, Bromid, Iodid, Tetrachlorozinkat, Methylsulfat, Trifluormethansulfonat oder Tetrafluoroborat als Gegenion, Isatin, 1-Methyl-isatin, 1-Allyl-isatin, 1-Hydroxymethyl-isatin, 5-Chlor-isatin, 5-Methoxy-isatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

Als CH-acide werden im allgemeinen solche Verbindungen angesehen, die ein an ein aliphatisches Kohlenstoffatom gebundenes Wasserstoffatom tragen, wobei aufgrund von elektronenziehenden Substituenten eine Aktivierung der entsprechenden Kohlenstoff-Wasserstoff-Bindung bewirkt wird. Unter CH-acide Verbindungen fallen erfindungsgemäß auch Enamine, die durch alkalische Behandlung von quaternierten N-Heterozyklen mit einer in Konjugation zum quartären Stickstoff stehenden CH-aciden Alkylgruppe entstehen.

Die CH-aciden Verbindungen der Komponente 2 sind bevorzugt ausgewählt aus der Gruppe bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazolium-p-toluolsulfonat, 2,3-Dimethyl-naphtho[1,2-d]thiazolium-p-toluolsulfonat, 3-Ethyl-2-methylnaphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 3-Methyl-1-phenylpyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methylbenzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methylbenzothiazolium-p-toluolsulfonat, 1-Ethyl-4-methyl-chinolinium-p-toluolsulfonat, 1-Ethyl-2-methylchinolinium-p-toluolsulfonat, und 1,2,3-Trimethylchinoxalinium-p-toluolsulfonat.

Bevorzugte primäre oder sekundäre aromatische Amine der Komponente 2 sind ausgewählt aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethol, 4-Amino-3-methylphenol, 2-(2,5-Diaminophenyl)-ethanol, 2,4-Diaminophenoxyethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Methylaminophenol, 2-Methyl-5-aminophenol, 3-Methyl-4-aminophenol, 2-Methyl-5-(2-hydroxyethylamino)phenol, 3-Amino-2-chlor-6-methylphenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methyl-benzol, 1-Hydroxy-2-amino-6-methyl-benzol, 2-Amino-5-acetamidophenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxy-benzoesäure, 4-Amino-3-hydroxy-benzoesäure, 2-Aminobenzolsulfonsäure, 3-Aminobenzolsulfonsäure, 4-Aminobenzolsulfönsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxybenzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr. 7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-nitrobenzol (HC Violet Nr. 1), 1-Amino-2-nitro-4-[(2,3-dihydroxypropyl)amino]-5-chlor-benzol (HC Red Nr. 10), 2-(4-Amino-2-nitroanilino)-benzoesäure, 6-Nitro-2,5-diaminopyridin, 2-Amino-6-chlor-4-nitrophenol, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydro-xy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hydroyethylamino)-2-nitrobenzol, aromatische Aniline bzw. Phenole mit einem weiteren aromatischen Rest, wie sie in der Formel II dargestellt sind 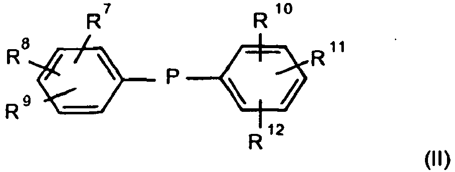 in der
- R⁷ für eine Hydroxy- oder eine Aminogruppe, die durch C₁₋₄-Alkyl-, C₁₋₄-Hydroxyalkyl-, C₁₋₄-Alkoxy- oder C₁₋₄-Alkoxy-C₁₋₄-alkylgruppen substituiert sein kann, steht,
- R⁸, R⁹, R¹⁰, R¹¹ und R¹² unabhängig voneinander für ein Wasserstoffatom, eine Hydroxy- oder eine Aminogruppe, die durch C₁-C₄-Alkyl-, C₁-C₄-Hydroxyalkyl, C₁-C₄-Alkoxy-, C₁-C₄-Amirioalkyl- oder C₁-C₄-Alkoxy-C₁-C₄-alkylgruppen substituiert sein kann, stehen, und
- P für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfoxy-, Sulfonyl- oder Iminogruppe, ein Sauerstoff- oder Schwefelatom, oder eine Gruppe mit der Formel III

   -Q'-(CH₂-Q-CH₂-Q")ₒ- (III)

   in der
   - Q eine direkte Bindung, eine CH₂- oder CHOH-Gruppe bedeutet,
   - Q' und Q" unabhängig voneinander für ein Sauerstoffatom, eine NR¹³-Gruppe, worin R¹³ ein Wasserstoffatom, eine C₁₋₄-Alkyl- oder eine Hydroxy-C₁₋₄-alkylgruppe, wobei auch beide Gruppen zusammen mit dem Restmolekül einen 5-, 6- oder 7-Ring bilden können, bedeutet, die Gruppe O-(CH₂)ₚ-NH oder NH-(CH₂)ₚ-O, worin p und p' 2 oder 3 sind, stehen und
   - o eine Zahl von 1 bis 4 bedeutet,
wie beispielsweise 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-amino-phenylamino)propan, , 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan.

Die vorgenannten Verbindungen können sowohl in freier Form als auch in Form ihrer physiologisch verträglichen Salze, insbesondere als Salze anorganischer Säuren, wie Salz- oder Schwefelsäure, eingesetzt werden.

Geeignete stickstoffhaltige heterocyclische Verbindungen sind z. B. 2-Aminopyridin, 3-Aminopyridin, 4-Aminopyridin, 2-Amino-3-hydroxy-pyridin, 2,6-Diamino-pyridin, 2,5-Diaminopyridin, 2-(Aminoethylamino)-5-aminopyridin, 2,3-Diamino-pyridin, 2-Dimethylamino-5-aminopyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 2,4-Dihydroxy-5,6-diaminopyrimidin, 4,5,6-Triaminopyrimidin, 4-Hydroxy-2,5,6-triaminopyrimidin, 2-Hydroxy-4,5,6-triaminopyrimidin, 2,4,5,6Tetraaminopyrimidin, 2-Methylamino-4,5,6-triaminopyrimidin, 2,4-Diaminopyrimidin, 4,5-Diaminopyrimidin, 2-Amino-4-methoxy-6-methyl-pyrimidin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 4-Aminochinaldin, 2-Aminonicotinsäure, 6-Aminoniootinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indol- und Indolinderivaten, wie 4-Aminoindol, 5-Aminoindol, 6-Aminoindol, 7-Aminoindol, 5,6-Dihydroxyindol, 5,6-Dihydroxyindolin und 4-Hydroxyindolin. Weiterhin als heterocyclische Verbindungen können erfindungsgemäß die in der DE-U1-299 08 573 offenbarten Hydroxypyrimidine eingesetzt werden. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Geeignete aromatische Hydroxyverbindungen sind z. B. 2-Methylresorcin, 4-Methylresorcin, 5-Methylresorcin, 2,5-Dimethylresorcin, Resorcin, 3-Methoxyphenol, Brenzkatechin, Hydrochinon, Pyrogallol, Phloroglucin, Hydroxyhydrochinon, 2-Methoxyphenol, 3-Methoxyphenol, 4-Methoxyphenol, 3-Dimethylaminophenol, 2-(2-Hydroxyethyl)phenol, 3,4-Methylendioxyphenol, 2,4-Dihydroxybenzoesäure, 3,4-Dihydroxybenzoesäure, 2,4-Dihydroxy-phenylessigsäure, 3,4-Dihydroxy-phenylessigsäure, Gallussäure, 2,4,6-Trihydroxybenzoesäure, 2,4,6-Trihydroxyacetophenon, 2-Chlorresorcin, 4-Chlorresorcin, 1-Naphthol, 1,5-Dihydroxynaphthalin, 2,3-Dihydroxynaphthalin, 2,7-Dihydroxynaphthalin, 6-Dimethylamino-4-hydroxy-2-naphthalinsulfonsäure und 3,6-Dihydroxy-2,7-naphthalinsulfonsäure.

Die Verbindungen der Komponente 1 und die Verbindungen der Komponente 2 werden vorzugsweise in den kosmetischen Mitteln jeweils in einer Menge von 0,03 bis 65 mmol, insbesondere von 1 bis 40 mmol, bezogen auf 100 g des gesamten Nuanciermittels, verwendet. Das molare Verhältnis von der Verbindung der Komponente 1 und der Verbindung der Komponente 2 kann im Bereich von 0,5 bis 2,0 liegen, wobei vorzugsweise äquimolare Mengen eingesetzt werden. Das anwendungsbereite Mittel wird bei getrennter Lagerung der Komponenten 1 und 2 unmittelbar vor der Anwendung durch Mischen hergestellt.

Als Vorstufen naturanaloger Farbstoffe werden bevorzugt als Oxidationsfarbstoffvorprodukt vom Entwicklertyp solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (IVa), 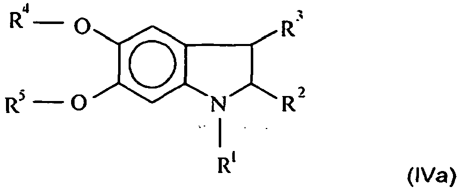 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IVb), 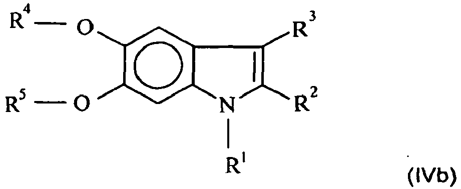 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann.
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Bevorzugte direktziehende Farbstoffe, die in den kosmetischen Mitteln als farbverändernde Komponente Verwendung finden, sind Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die kosmetischen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

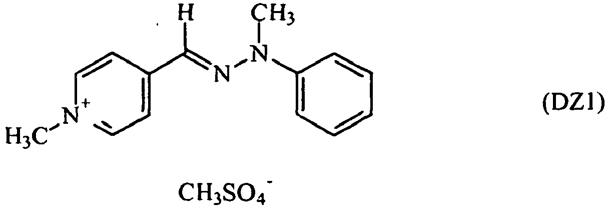

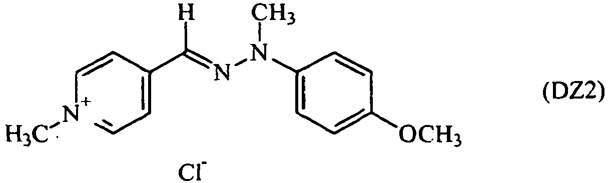

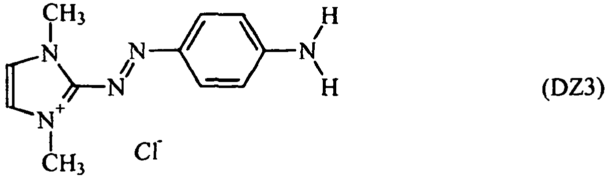

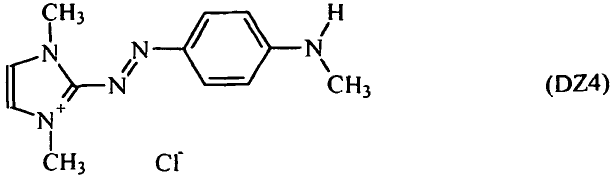

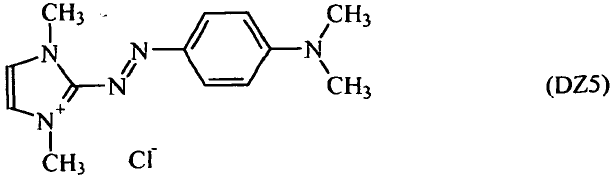

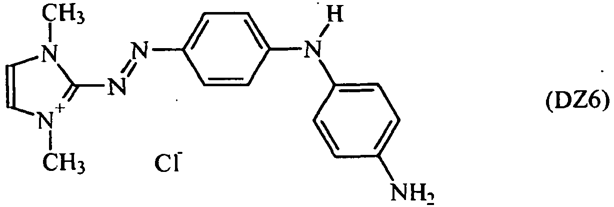

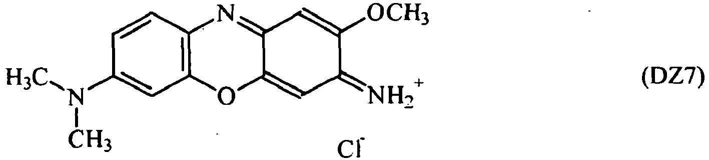

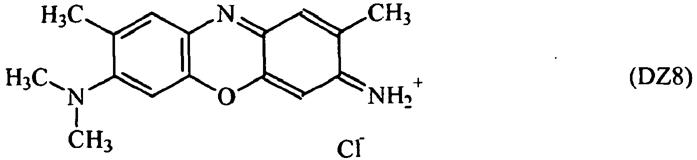

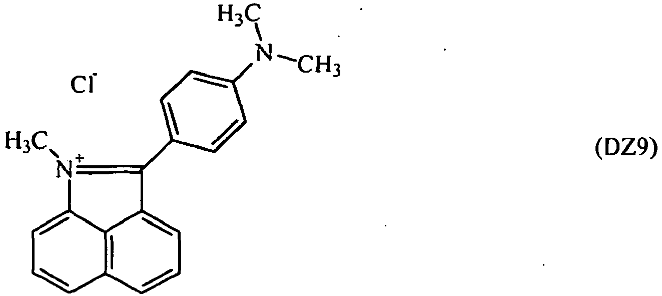

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die kosmetischen Mittel enthalten die direktziehenden Farbstoffe bevorzugt in einer Menge von 0,01 bis 20 Gew.-%, bezogen auf das anwendungsbereite Mittel.

Weiterhin können die erfindungsgemäßen kosmetischen Mittel auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Velmehr können in den kosmetischen Mitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die erfindungsgemäßen Mittel enthalten als farbverändernde Komponente (e) zusätzlich mindestens ein Oxidationsmittel. Desweiteren ist es bevorzugt, wenn die erfindungsgemäßen Mittel zusätzlich zu den farbverändemden Komponenten (a) bis (d) jeweils mindestens ein Oxidationsmittel enthalten.

Die Oxidationsmittel im Sinne der Erfindung sind von Luftsauerstoff verschieden und besitzen ein solches Oxidationspotenzial, das es ermöglicht, das natürliche Farbpigment Melanin oxidativ aufzuhellen und/oder ein Oxidationsfarbstoffvorprodukt vom Entwicklertyp zu oxidieren.

Als Oxidationsmittel kommt bevorzugt Wasserstoffperoxyd und/oder mindestens ein Anlagerungsprodukt davon, insbesondere an anorganische oder organische Verbindungen, wie beispielsweise Natriumperborat, Natriumpercarbonat, Magnesiumpercarbonat, Natriumpercarbamid, Polyvinylpyrrolidon·n H₂O₂ (n ist eine positive ganze Zahl größer 0), Harnstoffperoxid und Melaminperoxid in Frage.

Erfindungsgemäß kann das oxidative kosmetische Mittel auch zusammen mit einem Katalysator auf das Haar aufgebracht werden, der die Oxidation des Substrats, wie beispielsweise Oxidationsfarbstoffvorprodukte oder Melanin, aktiviert. Solche Katalysatoren sind z.B. Metallionen, Iodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung einer Färbung beschleunigt als auch die Farbnuance gezielt beeinflusst werden.

Geeignete Enzyme sind z.B. Peroxidasen, die die Wirkung geringer Mengen an Wasserstoffperoxyd deutlich verstärken können. Weiterhin sind solche Enzyme erfindungsgemäß geeignet, die mit Hilfe von Luftsauerstoff *in situ* geringe Mengen Wasserstoffperoxyd erzeugen und auf diese Weise die Oxidation der Farbstoffvorprodukte biokatalytisch aktivierten. Besonders geeignete Katalysatoren für die Oxidation von Farbstoffvorprodukten sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das Oxidationsmittel ist bevorzugt in einer Menge von 1,0 bis 10 Gew.-%, insbesondere von 3,0 bis 10,0 Gew.-%, jeweils bezogen auf das Gewicht des anwendungsbereiten Mittels, in dem oxidativen kosmetischen Mittel enthalten.

Das eigentliche oxidative Färbemittel wird bei getrennter Lagerung der Farbstoffvorprodukte und des Oxidationsmittels unmittelbar vor der Anwendung durch Mischen hergestellt. In einer bevorzugten Ausführungsform wird daher das kosmetische Mittel vor der Applikation aus einer Zusammensetzung, enthaltend in einem kosmetischen Träger mindestens eine farbverändernde Komponente, und einer weiteren Zusammensetzung, enthaltend in einem kosmetischen Träger mindestens ein Oxidationsmittel, gemischt. Dabei ist es wiederum bevorzugt, wenn die Farbstoffvorprodukte gemeinsam mit den Verbindungen der Formel (I) und den erfindungsgemäßen organischen Disulfidverbindungen getrennt vom Oxidationsmittel konfektioniert werden.

Bei einer Anwendung von Oxidationsmitteln wird die anwendungsfertige Zubereitung zweckmäßigerweise unmittelbar vor der Anwendung durch Mischung einer Zusammensetzung, enthaltend das Oxidationsmittel mit der Zusammensetzung, enthaltend die farbverändernden Komponenten, hergestellt. Das dabei entstehende gebrauchsfertige Haarpräparat sollte bevorzugt einen pH-Wert im Bereich von 6 bis 12, insbesondere von pH 7,5 bis 10, aufweisen.

Für eine Farbveränderung mittels Aufhellung bzw. Bleichung der Haare wird bevorzugt in den erfindungsgemäßen kosmetischen Mitteln zusätzlich zum Oxidationsmittel mindestens ein Bleichverstärker eingesetzt.

Bleichverstärker werden bevorzugt in Blondiermitteln zur Steigerung der Blondierwirkung eines Oxidationsmittels, insbesondere des Wasserstoffperoxyds, eingesetzt

Als Bleichverstärker können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat und 2,5-Diacetoxy-2,5-dihydrofuran.

Als Bleichverstärker können erfindungsgemäß bevorzugt Carbonatsalze, bzw. Hydrogencarbonatsalze eingesetzt werden. Diese sind bevorzugt ausgewählt aus der Gruppe der Ammonium-, Alkali- (insbesondere Natrium- und Kalium-), sowie Erdalkali- (insbesondere Calcium-), -carbonatsalze bzw. -hydrogencarbonatsalze. Besonders bevorzugte Carbonat-, bzw. Hydrogencarbonatsalze sind Ammoniumhydrogencarbonat, Ammoniumcarbonat, Natriumhydrogencarbonat, Dinatriumcarbonat, Kaliumhydrogencarbonat, Dikaliumcarbonat und Calciumcarbonat. Diese besonders bevorzugten Salze können allein oder in deren Mischungen von mindestens zwei Vertretern als Bleichverstärker verwendet werden.

Als Bleichverstärker vom Typ der Monoalkylcarbonate und deren Derivate werden bevorzugt mindestens ein Kohlensäuremonoester und/oder mindestens ein Kohlensäuremonoamid in dem erfindungsgemäßen Verfahren verwendet. Bevorzugt verwendbare Kohlensäuremonoester sind die Kohlensäuremonoester der Formel (V), 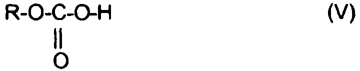 in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht.

In Formel (V) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sutfonsäuregruppen oder Halogene in Frage kommen. Weitere bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁-C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugt verwendete Zusammensetzungen sind dadurch gekennzeichnet, daß der Rest R in Formel (V) ausgewählt ist aus Methyl-, Ethyl-, n-Propyl-, iso-Propyl-, n-Butyl-, iso-Butyl-, *tert*-Butyl- sowie Hydroxymethyl- und Hydroxyethyl-Resten.

Alternativ zum Kohlensäuremonoester oder in Verbindung mit ihm können in den wasserfreien Zusammensetzungen Kohlensäuremonoamide als Bleichverstärker eingesetzt werden. Hier ist es erfindungsgemäß bevorzugt, mindestens ein Kohlensäuremonoamid der Formel (VI) zu verwenden, 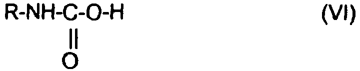 in der R für einen gesättigten oder ungesättigten, geradkettigen, verzweigten, oder cyclischen, substituierten oder unsubstituierten Kohlenwasserstoffrest, oder eine substituierte oder unsubstituierte Arylgruppe bzw. einen substituierten oder unsubstituierten Heterocyclus steht.

In Formel (VI) steht R vorzugsweise für einen substituierten oder unsubstituierten, geradkettigen oder verzweigten Alkyl-, Alkenyl- oder Alkinylrest, wobei als Substituenten bevorzugt Hydroxy-, Amino-, Nitro-, Sulfonsäuregruppen oder Halogene in Frage kommen. Weitere Bevorzugte Reste R sind Phenyl- und Benzylreste sowie weiter substituierte Vertreter. Besonders bevorzugt steht R für eine C₁₋₆-Alkylgruppe. Beispiele für erfindungsgemäße C₁-C₆-Alkylgruppen sind die Gruppen Methyl, Ethyl, n-Propyl, Isopropyl, n-Butyl, sec-Butyl, iso-Butyl, tert-Butyl, Pentyl, iso-Pentyl und Hexyl.

Erfindungsgemäß besonders bevorzugte Bleichverstärker der Formel (VI) sind dadurch gekennzeichnet, daß der Rest R in Formel (VI) ausgewählt ist aus Methyl-, Ethyl-, n-Propyl-, iso-Propyl-, n-Butyl-, iso-Butyl-, *tert*-Butyl- sowie Hydroxymethyl- und Hydroxyethyl-Resten.

Das acide H-Atom des Kohlensäuremonoesters bzw. -monoamids kann auch in neutralisierter Form vorliegen, d.h. es können erfindungsgemäß auch Salze von Kohlensäuremonoestern bzw. Kohlensäuremonoamiden eingesetzt werden. Hier sind erfindungsgemäß Kohlensäuremonoester bzw. das Kohlensäuremonoamide bevorzugt, die in ganz oder teilweise neutralisierter Form, vorzugsweise in Form des Alkamimetall-, Ammonium-, Erdalkalimetall- oder Aluminiumsalzes und insbesondere in Form seines Natriumsalzes, vorliegen.

Als Bleichverstärker vom Typ der Silylcarbonate und deren Derivate werden bevorzugt mindestens ein Silylcarbonat und/oder mindestens ein Silylcarbamat in die erfindungsgemäßen Zusammensetzungen eingearbeitet. Bevorzugt werden Silylcarbonate gemäß Formel (VII) eingesetzt, 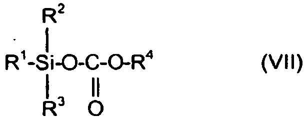 in der die Reste R¹, R² und R³ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstituierte Arylgruppe bzw. einen substituierten oder unsubstituierten Heterocyclus oder für ein Halogen, eine substituierte oder unsubstituierte Hydroxy-, Oxo-, Aminogruppen stehen und der Rest R⁴ für eine chemische Bindung zum Si-Atom oder zu einem der Reste R¹, R² oder R³, ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silyl- oder Aluminylgruppe oder für eine substituierte oder unsubstituierte Arylgruppe bzw. einen substituierten oder unsubstituierten Heterocyclus steht.

Bevorzugte Reste R¹, R² und R³ in der oben genannten Formel (VII) sind substituierte oder unsubstituierte, geradkettige oder verzweigte Alkylreste. Unter diesen sind die Alkylreste mit 1 bis 5 Kohlenstoffatomen sowie die Hydroxyalkylreste bevorzugt, so daß bevorzugte erfindungsgemäße wasserfreie Zusammensetzungen dadurch gekennzeichnet sind, daß die Reste R¹, R² und R³ in Formel (VII) ausgewählt sind aus Methyl-, Ethyl-, n-Propyl-, iso-Propyl-, n-Butyl-, iso-Butyl-, *tert*-Butyl- sowie Hydroxymethyl- und Hydroxyethyl-Resten.

Bevorzugte Reste R⁴ in der oben genannten Formel (VII) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Als Bleichverstärker kann mindestens ein Silylcarbamat der Formel (VIII) in der erfindungsgemäßen wasserfreien Zusammensetzung enthalten sein, 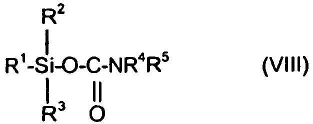 wobei die Reste R¹, R² und R³ unabhängig voneinander für ein Wasserstoffatom, einen gesättigten oder ungesättigten, geradkettigen, verzweigten, oder cyclischen, substituierten oder unsubstituierten Kohlenwasserstoffrest oder für eine Trialkylsilylgruppe, vorzugsweise eine Trimethylsilylgruppe oder für eine substituierte oder unsubstituierte Arylgruppe bzw. einen substituierten oder unsubstituierten Heterocyclus oder für ein Halogen, eine substituierte oder unsubstituierte Hydroxy-, Oxo-, Aminogruppen stehen und die Rest R⁴ und R⁵ unabhängig voneinander für eine chemische Bindung zum Si-Atom oder zu einem der Reste R¹, R² oder R³, ein Wasserstoffatom, einen gesättigten oder ungesättigten, geradkettigen, verzweigten, oder cyclischen, substituierten oder unsubstituierten Kohlenwasserstoffrest oder für eine substituierte oder unsubstituierte Silyl- oder Aluminylgruppe oder für eine substituierte oder unsubstituierte Arylgruppe bzw. einen substituierten oder unsubstituierten Heterocyclus stehen.

Bevorzugte Reste R¹, R² und R³ in der oben genannten Formel (VIII) sind substituierte oder unsubstituierte, geradkettige oder verzweigte Alkylreste. Unter diesen sind die Alkylreste mit 1 bis 5 Kohlenstoffatomen sowie die Hydroxyalkylreste bevorzugt, so daß bevorzugt verwendete Zusammensetzungen dadurch gekennzeichnet sind, daß die Reste R¹, R² und R³ in Formel (VIII) ausgewählt sind aus Methyl-, Ethyl-, n-Propyl-, iso-Propyl-, n-Butyl-, iso-Butyl-, *tert*-Butyl- sowie Hydroxymethyl- und Hydroxyethyl-Resten.

Bevorzugte Reste R⁴ und R⁵ in der oben genannten Formel (VIII) sind Wasserstoff, substituierte oder unsubstituierte, geradkettige oder verzweigte Alkylreste sowie Trialkylsilylreste. Unter diesen sind Wasserstoff, Methyl-, Ethyl-, tert.-Butyl- und Trimethylsilylreste bevorzugt.

Als weitere zusätzliche Bleichverstärker können in den erfindungsgemäßen Zusammensetzungen bevorzugt mindestens eine Verbindung ausgewählt aus Essigsäure, Milchsäure, Weinsäure, Zitronensäure, Salicylsäure und ortho-Phthalsäure, enthalten sein.

Bleichverstärker sind bevorzugt Peroxoverbindungen. Unter die erfindungsgemäß bleichverstärkenden Peroxoverbindungen fallen keine Anlagerungsprodukte von Wasserstoffperoxyd an andere Komponenten und auch nicht Wasserstoffperoxyd selbst. Die Auswahl der Peroxoverbindungen unterliegt darüber hinaus keinen Beschränkungen. Bevorzugte Peroxoverbindungen sind Peroxidisulfatsalze, Persulfatsalze, (insbesondere Ammoniumperoxidisulfat, Kaliumperoxidisulfat, Natriumperoxidisulfat, Ammoniumpersulfat, Kaliumpersulfat, Natriumpersulfat, Kaliumperoxidiphosphat) und Peroxide (wie Bariumperoxid und Magnesiumperoxid). Unter diesen Peroxoverbindungen, die auch in Kombination eingesetzt werden können, sind erfindungsgemäß die anorganischen Verbindungen bevorzugt. Besonders bevorzugt sind die Peroxidisulfate, insbesondere Ammoniumperoxidisulfat.

Die Bleichverstärker sind in den erfindungsgemäßen, kosmetischen Mitteln bevorzugt in Mengen von 5-30 Gew.-%, insbesondere in Mengen von 8-20 Gew.-%, enthalten.

Die kosmetischen Mittel der Erfindung enthalten, wenn sie als Bleichmittel fungieren, als bevorzugtes Alkalisierungsmittel mindestens eine Verbindung, ausgewählt aus Ammonium-, Alkalimetall- und Erdalkalimetallhydroxiden, -carbonaten, -hydrogencarbonaten, -hydroxycarbonaten, -metasilikaten und -carbamiden, sowie Alkaliphosphaten.

Die in dem erfindungsgemäßen Verfahren eingesetzten kosmetischen Mittel können weiterhin alle für solche Zubereitungen bekannten Wirk-, Zusatz- und Hilfsstoffe enthalten:

Das Nachwaschen mit einem Shampoo entfällt, wenn ein stark tensidhaltiger Träger verwendet wurde.

In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in den kosmetischen Mitteln alle für die Verwendung am menschlichen Körper geeigneten anionischen oberflächenaktiven Stoffe. Diese sind gekennzeichnet durch eine wasserlöslichmachende anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- anionische Alkyloligoglykoside bzw. anionische Alkenyloligoglykosid-Derivate, ausgewählt aus Alkyl- und/oder Alkenyl-Oligoglykosidcarboxylaten, -sulfaten, -phosphaten und/oder - isethionaten, die sich von Alkyl- und/oder Alkenyloligoglykosiden der allgemeinen Formel (IX) ableiten,

   R-O-(G)ₚ (IX)

   mit der Bedeutung
   R C₆₋₂₂-Alkyl oder C₆₋₂₂-Alkenyl,
   G Glykosideinheit, die sich von einem Zucker mit 5 oder 6 Kohlenstoffatomen ableitet,
   p Zahl von 1 bis 10,
   insbesondere das Laurylglucosidcarboxylat, wie es als Plantapon^{®} LGC von Cognis Deutschland erhältlich ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Besonders bevorzugt enthalten die efindungsgemäßen Mittel als (anionisches) Tensid zusätzlich mindestens eine Verbindung der Formel (X) und/oder der Formel (XI), 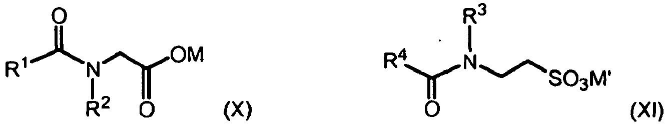 worin
- R¹: eine (C₇ bis C₃₀)-Alkylgruppe, (C₇ bis C₃₀)-Alkenylgruppe oder eine (C₇ bis C₃₀)-Hydroxyalkylgruppe bedeutet,
- R²: für eine (C₁ bis C₄)-Alkylgruppe oder eine (C₁ bis C₄)-Hydroxyalkylgruppe steht,
- R³: für ein Wasserstoffatom, eine (C₁ bis C₄)-Alkylgruppe oder eine (C₁ bis C₄)-Hydroxyalkylgruppe steht,
- R⁴: für eine (C₇ bis C₃₀)-Alkylgruppe, (C₇ bis C₃₀)-Alkenylgruppe oder eine (C₇ bis C₃₀)-Hydroxyalkylgruppe steht und
- M und M': unabhängig voneinander ein Wasserstoffatom oder ein Äquivalent eines ein- oder mehrwertigen Kations bedeuten.

Beispiele für erfindungsgemäße (C₁ bis C₄)-Alkylgruppen der Formeln (X) und (XI) sind Methyl, Ethyl, n-Propyl, Isopropyl, n-Butyl, sec-Butyl, iso-Butyl, tert-Butyl.
Beispiele für erfindungsgemäße (C₂ bis C₄)-Hydroxyalkylgruppen der Formeln (X) und (XI) sind 2-Hydroxyethyl, 3-Hydroxypropyl, 2-Hydroxypropyl und 4-Hydroxybutyl.
Beispiele für (C₇ bis C₃₀)-Alkenylgruppen der Formeln (X) und (XI) sind Heptadec-8-enyl, Heptadeca-8,11-dienyl, Heptadeca-8,11,14-trienyl, Nonadeca-4,7,10,13-tetraenyl, 12-Henicosenyl und Heptadeca-7,10,12-trienyl.
Die erfindungsgemäßen (C₇ bis C₃₀)-Alkylgruppen der Formeln (X) und (XI) können linear oder verzweigt sein.

Die Tenside der Formeln (X) und (XI) werden bevorzugt in Kombination, d.h. mindestens eine Verbindung gemäß Formel (X) gemeinsam mit mindestens einer Verbindung gemäß Formel (XI), verwendet.

Die Reste R¹ der Formel (X) und R⁴ der Formel (XI) stehen bevorzugt unabhängig voneinander für eine (C₉ bis C₂₃)-Alkylgruppe, (C₉ bis C₂₃)-Alkenylgruppe oder eine (C₉ bis C₂₃)-Hydroxyalkylgruppe, besonders bevorzugt unabhängig voneinander für einen Rest, ausgewählt aus der Liste, die gebildet wird, aus Nonyl, Undecyl, Tridecyl, Pentadecyl, Heptadecyl, Nonadecyl, Henicosanyl, 15-Methylhexadecyl, Heptadec-8-enyl, Heptadeca-8,11-dienyl, Nonadeca-4,7,10,13-tetraenyl, Heptadeca-8,11,14-trienyl und 11-Hydroxyheptadecyl.

Der Rest R² gemäß Formel (X) steht bevorzugt für einen Rest, ausgewählt aus der Gruppe, die gebildet wird aus Methyl, Ethyl, isopropyl, n-Propyl und 2-Hydroxyethyl. Besonders bevorzugt steht der besagte Rest R² für eine Methylgruppe.

Der Rest R³ gemäß Formel (XI) steht bevorzugt für einen Rest, ausgewählt aus der Gruppe, die gebildet wird aus Wasserstoffatom, Methyl, Ethyl, Isopropyl, n-Propyl und 2-Hydroxyethyl. Besonders bevorzugt steht R² der Formel (X) für ein Wasserstoffatom oder eine Methylgruppe.

Es ist erfindungsgemäß ganz besonders bevorzugt, wenn R² der Formel (X) und R³ der Formel (XI) beide gemeinsam für eine (C₁ bis C₄)-Alkylgruppe, insbesondere für eine Methylgruppe, stehen.

Wenn die Verbindungen der Formeln (X) bzw. (XI) als Säure vorliegen, bedeutet der Rest M bzw. der Rest M' ein Wasserstoffatom. Wenn die Verbindungen der Formeln (X) bzw. (XI) als Salz vorliegen, stehen M bzw. M' für ein Äquivalent eines ein- oder mehrwertigen Kations. Für die ein-oder mehrwertigen Kationen gilt mutatis mutandis das für die Kationen der Formel (ii-1) bzw. (ii-2) Gesagte.

Die Verbindungen der Formel (X) werden bevorzugt ausgewählt aus mindestens einem Vertreter aus der Gruppe, die gebildet wird, aus N-Lauroylsarcosin, N-Myristoylsarcosin, N-Palmitoylsarcosin, N-Oleylsarcosin, N-Cocoylsarcosin (wobei hier eine Gruppe von Verbindungen vorliegt und Cocoyl der Zusammensetzung des Fettsäureschnitt des Kokosnussöls entspricht) und N-Palmkernsarcosin (wobei hier eine Gruppe von Verbindungen vorliegt und Palmkern der Zusammensetzung des Fettsäureschnitt des Palmkernöls entspricht), sowie aus den Salzen der vorgenannten N-Acylsarcosinderivate.

Die Verbindungen der Formel (XI) werden bevorzugt ausgewählt aus mindestens einem Vertreter aus der Gruppe, die gebildet wird, aus N-Dodecanoyl-N-methyltaurin, N-Octadecanoyl-N-methyltaurin, N-Hexadecanoyl-N-methyltaurin, N-Tetradecanoyl-N-methyltaurin, N-Oleyl-N-methyltaurin, N-Cocoyl-N-methyltaurin (wobei hier eine Gruppe von Verbindungen vorliegt und Cocoyl der Zusammensetzung des Fettsäureschnitt des Kokosnussöls entspricht), N-Palmkern-N-methyltaurin (wobei hier eine Gruppe von Verbindungen vorliegt und Palmkern der Zusammensetzung des Fettsäureschnitt des Palmkernöls entspricht), sowie aus den Salzen der vorgenannten N-Acyltaurinderivate.

Die Triglyceride des Kokosöls weisen folgende Fettsäurezusammensetzung auf:

| | |
|---|---|
| 45 bis 51 % | Laurinsäure |
| 16 bis 19 % | Tetradecansäure |
| 8 bis 10 % | Ölsäure |
| 9 bis 11 % | Palmitinsäure |
| 6 bis 9 % | Decansäure |
| 5 bis 8 % | Octansäure |

Die Triglyceride des Palmkernöls weisen folgende Fettsäurezusammensetzung auf:

| | |
|---|---|
| 47 bis 52 % | Laurinsäure |
| 16 bis 18 % | Tetradecansäure |
| 10 bis 18 % | Ölsäure |
| 6 bis 9 % | Palmitinsäure |
| 2 bis 3 % | Stearinsäure |
| 1 bis 3 % | Linolsäure |
| 3 bis 5 % | Decansäure |
| 1 bis 3 % | Octansäure |

Die erfindungsgemäßen Mittel enthalten die Verbindungen der Formel (X) und/oder der Formel (XI) bevorzugt in einer Menge von 0,1 bis 15 Gew.-%, besonders bevorzugt von 0,5 bis 10 Gew.-%, ganz besonders bevorzugt von 0,5 bis jeweils bezogen auf das Gewicht des anwendungsbereiten Mittels.

Nichtionogene Tenside enthalten als hydrophile Gruppe z. B. eine Polyolgruppe, eine Po-lyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)ₓ. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkornponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Weiterhin können, insbesondere als Co-Tenside, zwitterionische Tenside verwendet werden. Als zwitterionische Tenside werden solche oberflächenaktive Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acyl-aminopropyl-N,N-dimethylammoniumglycinate, beispielsweise das Kokosacylaminopropyl-dimethylammoniumglycinat, und 2-Alkyl-3-carboxylmethyl-3-hydroxy-ethyl-imidazoline mit jeweils 8 bis 18 C-Atomen in der Alkyl- oder Acylgruppe sowie das Kokos-acylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine - COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quatemium-27 und Quaternium-83 bekannten Imidazolium-Verbindungen. Die langen Alkylketten der oben genannten Tenside weisen bevorzugt 10 bis 18 Kohlenstoffatome auf.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quatemierte Estersalze von Fettsäuren mit Triethanolamin, quatemierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Weitere erfindungsgemäß verwendbare kationische Tenside stellen die quatemisierten Proteinhydrolysate dar.

Erfindungsgemäß ebenfalls geeignet sind kationische Silikonöle wie beispielsweise die im Handel erhältlichen Produkte Q2-7224 (Hersteller: Dow Coming; ein stabilisiertes Trime-thylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quatemium-80).

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "nor-malen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder - alkoholate als Katalysatoren verwendet werden. Die Verwendung von Produkten mit eingeengter Homologenverteilung kann bevorzugt sein.

Die erfindungsgemäßen Mittel können zusätzlich mindestens ein Silikon enthalten. Die Silikone, wenn sie in den erfindungsgemäßen Mitteln zugegen sind, vorzugsweise in Mengen von 0,05 bis 5 Gew.-%, vorzugsweise von 0,2 bis 5 Gew.-%, jeweils bezogen auf das anwendungsbereite Mittel, enthalten.

Insbesondere bevorzugt werden die Silikone ausgewählt, aus mindestens einem Vertreter aus der Liste, die gebildet wird aus:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere .organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Silikonpolymeren mit nicht silikonhaltigem, organischen Grundgerüst, die aus einer organischen Hauptkette bestehen, welche aus organischen Monomeren gebildet wird, die kein Silikon enthalten, auf die in der Kette sowie gegebenenfalls an mindestens einem Kettenende mindestens ein Polysiloxanmakromer gepfropft wurde;
(v) gepfropften Silikonpolymeren mit Polysiloxan-Grundgerüst, auf das nicht silikonhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silikon enthält, wie beispielsweise das unter der INCI-Bezeichnung Bis-PEG/PPG-20/20 Dimethicone vertriebene Handelsprodukt Abil B 8832 der Firma Degussa;
(vi) oder deren Gemischen.

Besonders bevorzugte erfindungsgemäße kosmetische oder dermatologische Zubereitungen sind dadurch gekennzeichnet, dass sie mindestens ein Silikon der Formel (Si-1)

(CH₃)₃Si-[O-Si(CH₃)₂]ₓ-O-Si(CH₃)₃ (Si-1),

enthalten, in der x für eine Zahl von 0 bis 100, vorzugsweise von 0 bis 50, weiter bevorzugt von 0 bis 20 und insbesondere 0 bis 10, steht.

Die erfindungsgemäß bevorzugten kosmetischen oder dermatologischen Zubereitungen enthalten ein Silikon der vorstehenden Formel (Si-1). Diese Silikone werden nach der INCI-Nomenklatur als Dimethicone bezeichnet. Es werden im Rahmen der vorliegenden Erfindung als Silikon der Formel (Si-1) vorzugsweise die Verbindungen:
(CH₃)₃Si-O-Si(CH₃)₃
(CH₃)₃Si-O-(CH₃)₂Si-O-Si(CH₃)₃
(CH₃)₃Si-[O-(CH₃)₂Si]₂-O-Si(CH₃)₃
(CH₃)₃Si-[O-(CH₃)₂Si]₃-O-Si(CH₃)₃
(CH₃)₃Si-[O-(CH₃)₂Si]₄-O-Si(CH₃)₃
(CH₃)₃Si-[O-(CH₃)₂Si]₅-O-Si(CH₃)₃
(CH₃)₃Si-[O-(CH₃)₂Si]₆-O-Si(CH₃)₃
(CH₃)₃Si-[O-(CH₃)₂Si]₇-O-Si(CH₃)₃
(CH₃)₃Si-[O-(CH₃)₂Si]₈-O-Si(CH₃)₃
(CH₃)₃Si-[O-(CH₃)₂Si]₉-O-Si(CH₃)₃
(CH₃)₃Si-[O-(CH₃)₂Si]₁₀-O-Si(CH₃)₃
(CH₃)₃Si-[O-(CH₃)₂Si]₁₁-O-Si(CH₃)₃
(CH₃)₃Si-[O-(CH₃)₂Si]₁₂-O-Si(CH₃)₃
(CH₃)₃Si-[O-(CH₃)₂Si]₁₃-O-Si(CH₃)₃
(CH₃)₃Si-[O-(CH₃)₂Si]₁₄-O-Si(CH₃)₃
(CH₃)₃Si-[O-(CH₃)₂Si]₁₅-O-Si(CH₃)₃
(CH₃)₃Si-[O-(CH₃)₂Si]₁₆-O-Si(CH₃)₃
(CH₃)₃Si-[O-(CH₃)₂Si]₁₇-O-Si(CH₃)₃
(CH₃)₃Si-[O-(CH₃)₂Si]₁₈-O-Si(CH₃)₃
(CH₃)₃Si-[O-(CH₃)₂Si]₁₉-O-Si(CH₃)₃
(CH₃)₃Si-[O-(CH₃)₂Si]₂₀-O-Si(CH₃)₃
eingesetzt, wobei (CH₃)₃Si-O-Si(CH₃)₃, (CH₃)₃Si-O-(CH₃)₂Si-O-Si(CH₃)₃ und/oder (CH₃)₃Si-[O-(CH₃)₂Si]₂-O-Si(CH₃)₃ besonders bevorzugt sind.

Selbstverständlich können auch Mischungen der o.g. Silikone in den bevorzugten erfindungsgemäßen Mitteln enthalten sein.

Bevorzugte erfindungsgemäß einsetzbare Silikone weisen bei 20°C Viskositäten von 0,2 bis 2 mm²s⁻¹ auf, wobei Silikone mit Viskositäten von 0,5 bis 1 mm²s⁻¹ besonders bevorzugt sind.

Besonders bevorzugte efindungsgemäße Mittel enthalten ein oder mehrere aminofunktionelle Silikone. Solche Silikone können z.B. durch die Formel (Si-2)

M(RₐQ_{b}SiO_{(4-a-b/2})ₓ(R_{c}SiO_{(4-c)/2})_{y}M (Si-2)

Beschrieben werden, wobei in der obigen Formel
- R: ein Kohlenwasserstoff oder ein Kohlenwasserstoffrest mit 1 bis etwa 6 Kohlenstoffatomen ist,
- Q: ein polarer Rest der allgemeinen Formel -R¹HZ ist, worin
- R¹: eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und
- Z: ein organischer, aminofunktioneller Rest ist, der mindestens eine aminofunktionelle Gruppe enthält;
- a: Werte im Bereich von etwa 0 bis etwa 2 annimmt,
- b: Werte im Bereich von etwa 1 bis etwa 3 annimmt,
- a + b: kleiner als oder gleich 3 ist, und
- c: eine Zahl im Bereich von etwa 1 bis etwa 3 ist, und
- x: eine Zahl im Bereich von 1 bis etwa 2.000, vorzugsweise von etwa 3 bis etwa 50 und am bevorzugtesten von etwa 3 bis etwa 25 ist, und
- y: eine Zahl im Bereich von etwa 20 bis etwa 10.000, vorzugsweise von etwa 125 bis etwa 10.000 und am bevorzugtesten von etwa 150 bis etwa 1.000 ist, und
- M: eine geeignete Silikon-Endgruppe ist, wie sie im Stande der Technik bekannt ist, vorzugsweise Trimethylsiloxy.

Nicht einschränkende Beispiele der in Formel (Si-2) durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen,-CH₂CH(CH₃)CH₂-, Phenylen, Naphthylen, -CH₂CH₂SCH₂CH₂-, -CH₂CH₂OCH₂-, -OCH₂CH₂-,-OCH₂CH₂CH₂-, -CH₂CH(CH₃)C(O)OCH₂-, -(CH₂)₃CC(O)OCH₂CH₂-, -C₆H₄C₆H₄-, -C₆H ₄CH₂C₆H₄-; und -(CH₂)₃C(O)SCH₂CH₂- ein.

Z ist gemäß Formel (Si-2) ein organischer, aminofunktioneller Rest, enthaltend mindestens eine funktionelle Aminogruppe. Eine mögliche Formel für besagtes Z ist NH(CH₂)_{z}NH₂, worin z eine ganze Zahl von größer gleich 1 ist. Eine andere mögliche Formel für besagtes Z ist -NH(CH₂)_{z}(CH ₂)_{zz}NH, worin sowohl z als auch zz unabhängig voneinander eine ganze Zahl von größer gleich 1 sind, wobei diese Struktur Diamino-Ringstrukturen umfasst, wie Piperazinyl. Besagtes Z ist am bevorzugtesten ein -NHCH₂CH₂NH₂-Rest. Eine andere mögliche Formel für besagtes Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q gemäß Formel (Si-2) ist am bevorzugtesten ein polarer aminofunktioneller Rest der Formel - CH₂CH₂CH₂NHCH₂CH₂NH₂.

In der Formel (Si-2) nimmt a Werte im Bereich von 0 bis 2 an, b nimmt Werte im Bereich von 2 bis 3 an, a + b ist kleiner als oder gleich 3, und c ist eine Zahl im Bereich von 1 bis 3. Das molare Verhältnis der RₐQ_{b} SiO_{(4-a-b)/2}-Einheiten zu den R_{c}SiO(_{4-c)/2}-Einheiten in Formel (Si-2) liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silikone der obigen Formel (Si-2) eingesetzt, dann können die verschiedenen variablen Substituenten in der obigen Formel bei den verschiedenen Silikonkomponenten, die in der Silikonmischung vorhanden sind, verschieden sein.

Bevorzugte efindungsgemäße kosmetische oder dermatologische Zubereitungen enthalten ein aminofunktionelles Silikon der Formel (Si-3)

R'ₐG₃₋ₐ-Si(OSiG₂)ₙ-(OSiG_{b}R'_{2-b})ₘ-O-SiG₃₋ₐ-R'ₐ (Si-3),

worin bedeutet:
- G: ist -H, eine Phenylgruppe, -OH, -O-CH₃, -CH₃, -O-CH₂CH₃, -CH₂CH₃, -O-CH₂CH₂CH₃, -CH₂CH₂CH₃, -O-CH(CH₃)₂, -CH(CH₃)₂, -O-CH₂CH₂CH₂CH₃, -CH₂CH₂CH₂CH₃, -O-CH₂CH(CH₃)₂, -CH₂CH(CH₃)₂, -O-CH(CH₃)CH₂CH₃, -CH(CH₃)CH₂CH₃, -O-C(CH₃)₃, -C(CH₃)₃ ;
- a: steht für eine Zahl zwischen 0 und 3, insbesondere 0;
- b: steht für eine Zahl zwischen 0 und 1, insbesondere 1,
- m und n: sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R': ist ein monovalenter Rest ausgewählt aus
-Q-N(R")-CH₂-CH₂-N(R")₂
-Q-N(R")₂
-Q-N⁺(R")₃A⁻
-Q-N⁺H(R")₂A⁻
-Q-N⁺H₂(R")A⁻
-Q-N(R")-CH₂-CH₂-N⁺R"H₂A⁻,
wobei jedes Q für eine chemische Bindung, -CH₂-, -CH₂-CH₂-, -CH₂CH₂CH₂-, -C(CH₃)₂-, -CH₂CH₂CH₂CH₂-, -CH₂C(CH₃)₂-, -CH(CH₃)CH₂CH₂- steht, R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃,-CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Erfindungsgemäß geeignet sind kationische Silikonöle wie beispielsweise die im Handel erhältliche Dow Corning 929 Emulsion (enthaltend ein hydroxylamino-modifiziertes Silikon, das als Amodimethicone bezeichnet wird), DC 2-2078 (Hersteller Dow Corning, INCI-Bezeichnung: Aminopropyl Phenyl Trimethicone), DC 5-7113 (Hersteller Dow Corning, INCI-Bezeichnung: Silicone Quaternium 16), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quatemium-80).

Besonders bevorzugte erfindungsgemäße Mittel sind dadurch gekennzeichnet, dass sie mindestens es ein aminofunktionelles Silikon der Formel (Si3-a) 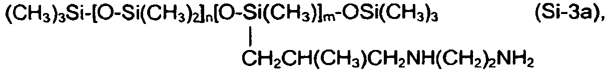 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Trimethylsilylamodimethicone bezeichnet und sind beispielsweise unter der Bezeichnung Q2-7224 (Hersteller: Dow Coming; ein stabilisiertes Trimethylsilylamodimethicon) erhältlich.

Besonders bevorzugt sind auch erfindungsgemäße Mittel, die mindestens ein aminofunktionelles Silikon der Formel (Si-3b) 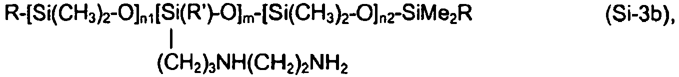 enthalten, worin
- R: für -OH, eine (gegebenenfalls ethoxylierte und/oder propoxylierte) (C₁ bis C₂₀)-Alkoxygruppe oder eine -CH₃-Gruppe steht,
- R': für -OH, eine (C₁ bis C₂₀)-Alkoxygruppe oder eine -CH₃-Gruppe und
- m, n1 und n2: Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese Silikone werden nach der INCI-Deklaration als Amodimethicone, bzw. als funktionalisierte Amodimethicone, wie beispielsweise Bis(C13-15 Alkoxy) PG Amodimethicone (beispielsweise als Handelsprodukt: DC 8500 der Firma Dow Corning erhältlich), Trideceth-9 PG-Amodimethicone (beispielsweise als Handelsprodukt Silcare Silicone SEA der Firma Clariant erhältlich) bezeichnet.

Unabhängig davon, welche aminofunktionellen Silikone eingesetzt werden, sind erfindungsgemäße kosmetische oder dermatologische Zubereitungen bevorzugt, die ein aminofunktionelles Silikon enthalten dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silikons. Sie kann durch Titration ermittelt und auch in der Einheit mg KOH/g angegeben werden.

Erfindungsgemäß bevorzugte kosmetische oder dermatologische Zubereitungen sind dadurch gekennzeichnet, dass sie, bezogen auf ihr Gewicht, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silikon(e) enthalten.

Auch die nach INCI als Cyclomethicone bezeichneten cyclischen Dimethicone sind erfindungsgemäß mit Vorzug einsetzbar. Hier sind erfindungsgemäße kosmetische oder dermatologische Zubereitungen bevorzugt, die mindestens ein Silikon der Formel (Si-4) 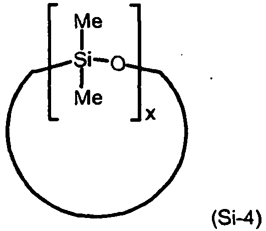 enthalten, in der x für eine Zahl von 0 bis 200, vorzugsweise von 0 bis 10, weiter bevorzugt von 0 bis 7 und insbesondere 0, 1, 2, 3, 4, 5 oder 6, steht.

Die vorstehend beschriebenen Silikone weisen ein Rückgrat auf, welches aus -Si-O-Si-Einheiten aufgebaut ist. Selbstverständlich können diese Si-O-Si-Einheiten auch durch Kohlenstoffketten unterbrochen sein. Entsprechende Moleküle sind durch Kettenverlängerungsreaktionen zugänglich und kommen vorzugsweise in Form von Silikon-in-Wasser-Emulsionen zum Einsatz.

Die erfindungsgemäß einsetzbaren Silikon-in-Wasser Emulsionen können nach bekannten Verfahren hergestellt werden, wie sie beispielsweise in US 5,998,537 und EP 0 874 017 A1 offenbart sind.

Zusammenfassend umfasst dieses Herstellungsverfahren die emulgierende Mischung von Komponenten, deren eine mindestens ein Polysiloxane enthält, deren andere mindestens ein Organosilikonmaterial enthält, das mit dem Polysiloxane in einer Kettenverlängerungsreaktion reagiert, wobei mindestens ein Metallion-enthaltender Katalysator für die Kettenverlängerungsreaktion, mindestens ein Tensid und Wasser zugegen sind.

Kettenverlängerungsreaktionen mit Polysiloxanen sind bekannt und können beispielsweise die Hydrosilylierungsreaktion umfassen, in der eine Si-H Gruppe mit einer aliphatisch ungesättigten Gruppe in Gegenwart eines Platin/Rhodium-Katalysators unter Bildung von Polysiloxanes mit einigen Si-(C)ₚ-Si Bindungen (p = 1-6) reagiert, wobei die Polysiloxane auch als Polysiloxane-Polysilalkylene-Copolymere bezeichnet werden.

Die Kettenverlängerungsreaktion kann auch die Reaktion einer Si-OH Gruppe (beispielsweise eines Hydroxy-terminierten Polysiloxans) mit einer Alkoxygruppe (beispielsweise Alkoxysilanen, Silikaten oder Alkoxysiloxanen) in Gegenwart eines metallhaltigen Katalysators unter Bildung von Polysiloxanen umfassen.

Die Polysiloxane, die in der Kettenverlängerungsreaktion eingesetzt werden, umfassen ein substantiell lineares Polymer der folgenden Struktur:

R-Si(R₂)-[-O-Si(R₂)-]ₙ-O-SiR₃

In dieser Struktur steht jedes R unabhängig voneinander für einen Kohlenwasserstoffrest mit bis zu 20 Kohlenstoffatomen, vorzugsweise mit 1 bis 6 C-Atomen, wie beispielsweise einer Alkylgruppe (beispielsweise Methyl, Ethyl, Propyl oder Butyl), eine Arylgruppe (beispielsweise Phenyl), oder die für die Kettenverlängerungsreaktion benötigte Gruppe ("reaktive Gruppe", beispielsweise Si-gebundene H-Atome, aliphatisch ungesättigte Gruppen wie Vinyl, Allyl oder Hexenyl, Hydroxy, Alkoxy wie Methoxy, Ethoxy oder Propoxy, Alkoxy-Alkoxy, Acetoxy, Amino usw.), mit der Maßgabe, dass durchschnittlich ein bis zwei reaktive Gruppen pro Polymer vorliegen, n ist eine positive Zahl > 1. Vorzugsweise ist eine Mehrzahl der reaktiven Gruppen, besonders bevorzugt > 90%, und insbesondere > 98% der reaktiven Gruppen, an den endständigen Si-Atomen im Siloxan gebunden. Vorzugsweise steht n für Zahlen, die Polysiloxane beschreiben, welche Viskositäten zwischen 1 und 1.000.000 mm²/s besitzen, besonders bevorzugt Viskositäten zwischen 1.000 und 100.000 mm²/s.

Die Polysiloxane können zu einem geringen Grad verzweigt sein (beispielsweise < 2 Mol-% der Siloxaneinheiten), bzw. sind die Polymere aber substantiell linear, besonders bevorzugt vollständig linear. Zudem können die Substituenten R ihrerseits substituiert sein, beispielsweise mit N-haltigen Gruppen (beispielsweise Aminogruppen), Epoxygruppen, S-haltige Gruppen, Sihaltige Gruppen, O-haltige Gruppen usw.. Vorzugsweise sind mindestens 80% der Reste R Alkylreste, besonders bevorzugt Methylgruppen.

Das Organosilikonmaterial, das mit dem Polysiloxan in der Kettenverlängerungsreaktion reagiert, kann entweder ein zweites Polysiloxan sein, oder ein Molekül, das als Kettenverlängerer agiert. Wenn das Organosilikonmaterial ein Polysiloxan ist, hat es die vorstehend erwähnte generelle Struktur. In diesen Fällen besitzt ein Polysiloxan in der Reaktion (mindestens) eine reaktive Gruppe, und ein zweites Polysiloxan besitzt (mindestens) eine zweite reaktive Gruppe, die mit der ersten reagiert.

Falls das Organosilikonmaterial ein Kettenverlängerungs-Agens umfasst, kann dies ein Material sein wie beispielsweise ein Silan, ein Siloxan (beispielsweise Disiloxane oder Trisiloxan) oder ein Silazan. So kann beispielsweise eine Zusammensetzung, die ein Polysiloxan gemäß der vorstehend beschriebenen generellen Struktur umfasst, welches mindestens eine Si-OH Gruppe aufweist, ketteverlängert werden, indem mit einem Alkoxysilan (beispielsweise einem Dialkoxysilan oder Trialkoxysilan) in Gegenwart von Zinn- oder Titan-haltigen Katalysatoren reagiert wird.

Die metallhaltigen Katalysatoren in der Kettenverlängerungsreaktion sind meist spezifisch für eine bestimmte Reaktion. Solche Katalysatoren sind im Stand der Technik bekannt und enthalten beispielsweise Metalle wie Platin, Rhodium, Zinn, Titan, Kupfer, Blei, etc.. In einer bevorzugten Kettenverlängerungsreaktion wird ein Polysiloxan mit mindestens einer aliphatisch ungesättigten Gruppe, vorzugsweise einer Endgruppe, mit einem Organosilikonmaterial in Gegenwart eines Hydrosilylierungskatalysators zur Reaktion gebracht, das ein Siloxan oder Polysiloxan mit mindestens einer (vorzugsweise endständigen) Si-H Gruppe ist. Das Polysiloxan besitzt mindestens eine aliphatisch ungesättigte Gruppe und genügt der allgemeinen oben angegeben Formel, in der R und n wie vorstehend definiert sind, wobei im Durchschnitt zwischen 1 und 2 Gruppen R eine aliphatisch ungesättigte Gruppe pro Polymer besitzen. Repräsentative aliphatisch ungesättigte Gruppen sind beispielsweise Vinyl, Allyl, Hexenyl und Cyclohexenyl oder eine Gruppe R²CH=CHR³, in der R² für eine divalente aliphatische an das Silicium gebundene Kette und R³ für ein Wasserstoffatom oder eine Alkylgruppe steht. Das Organosilikonmaterial mit mindestens einer Si-H Gruppe hat vorzugsweise die oben genannte Struktur, in der R und n wie vorstehend definiert sind und wobei im Durchschnitt zwischen 1 und 2 Gruppen R ein Wasserstoff bedeuten und n 0 oder eine positive ganze Zahl ist.

Dieses Material kann ein Polymer oder ein niedermolekulares Material wie ein Siloxan sein (beispielsweise ein Disiloxane oder ein Trisiloxan).

Das Polysiloxan mit mindestens einer aliphatisch ungesättigten Gruppe und das Organosilikonmaterial mit mindestens einer Si-H Gruppe reagieren in Gegenwart eines Hydrosilylierungskatalysators. Solche Katalysatoren sind aus dem Stand der Technik bekannt und umfassen beispielsweise Platin- und Rhodium-enthaltende Materialien. Die Katalysatoren können jede bekannte Form annehmen, beispielsweise auf Trägermaterialien (wie beispielsweise Silica Gel oder Aktivkohle) aufgebrachtes Platin oder Rhodium oder andere geeignete Compounds wie Platinchlorid, Salze von Platin- oder Chloroplatinsäuren. Ein wegen der guten Dispergierbarkeit in Organosilikonsystemen und der geringen Farbveränderung bevorzugter Katalysator ist Chloroplatinsäure entweder als kommerziell verfügbares Hexahydrat oder in wasserfreier Form.

Bei einer weiteren bevorzugten Kettenerweiterungsreaktion wird ein Polysiloxan mit mindestens einer Si-OH Gruppe, vorzugsweise einer Endgruppe, mit einem Organosilikonmaterial zu Reaktion gebracht, das mindestens eine Alkoxygruppe besitzt, vorzugsweise ein Siloxan mit mindestens einer Si-OR Gruppe oder ein Alkoxysilan mit mindestens zwei Alkoxygruppen. Hierbei wird als Katalysator wieder ein metallhaltiger Katalysator eingesetzt.

Für die Reaktion einer Si-OH Gruppe mit einer Si-OR Gruppe existieren viele literaturbekannte Katalysatoren, beispielsweise Organometallverbindungen wie Organozinnsalze, Titanate oder Titanchelate bzw. -komplexe. Beispiele umfassen Zinn-octoat, Dibutylzinn-dilaurat, Dibutylzinndiacetat, Dimethyltinn-dineodecanoat, Dibutylzinn-dimethoxid, Isobutylzinn-triceroat, Dimethylzinn-dibutyrat, Dimethylzinn-dineodecanoat, Triethylzinn-tartrat, Zinnoleat, Zinnnaphthenat, Zinnbutyrat, Zinnacetat, Zinnbenzoat, Zinnsebacat, Zinnsuccinat, Tetrabutyltitanat, Tetraisopropyltitante, Tetraphenyltitanat, Tetraoctadecyltitanat, Titannaphthanat, Ethyltriethanolamin-Titanat, Titani-diisopropyl-diethyl-acetoacetat, Titan-diisopropoxydiacetyl-acetonat und Titani-tetra-Alkoxide, bei denen das Alkoxid Butoxy oder Propoxy ist.

Erfindungsgemäß ebenfalls bevorzugte Mittel sind dadurch gekennzeichnet, dass sie mindestens ein Silikon der Formel (Si-5)

R₃Si-[O-SiR₂]ₓ-(CH₂)ₙ-[O-SiR₂]_{y}-O-SiR₃ (Si-5),

enthalten, in der R für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃, steht, x bzw. y für eine Zahl von 0 bis 200, vorzugsweise von 0 bis 10, weiter bevorzugt von 0 bis 7 und insbesondere 0, 1, 2, 3, 4, 5 oder 6, stehen, und n für eine Zahl von 0 bis 10, bevorzugt von 1 bis 8 und insbesondere für 2, 3, 4, 5, 6 steht.

Mit Vorzug sind die Silikone wasserlöslich. Erfindungsgemäß bevorzugte Mittel der Ausführungsform mit einem Silikon sind dadurch gekennzeichnet, dass das Silikon wasserlöslich ist.

Entsprechende hydrophile Silikone werden beispielsweise aus den Verbindungen der Formeln (Si-6) und/oder (Si-7) ausgewählt. Insbesondere bevorzugte wasserlösliche Tenside auf Silikonbasis sind ausgewählt aus der Gruppe der Dimethiconcopolyole die bevorzugt alkoxyliert, insbesondere polyethoxyliert oder polypropoxyliert sind.

Unter Dimethiconcopolyolen werden erfindungsgemäß bevorzugt Polyoxyalkylen-modifizierte Dimethylpolysiloxane der allgemeinen Formeln (Si-6) oder (Si-7) verstanden: 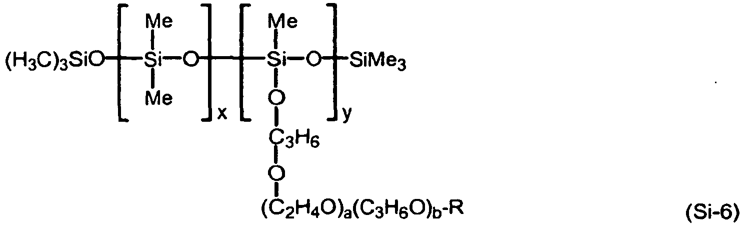 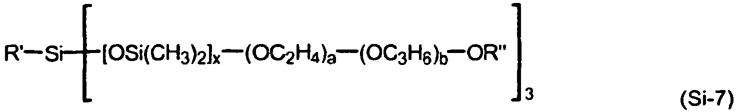 worin
- der Rest R steht für ein Wasserstoffatom, eine Alkylgruppe mit 1 bis 12 C-Atomen, eine Alkoxygruppe mit 1 bis 12 C-Atomen oder eine Hydroxylgruppe,
- die Reste R' und R" bedeuten Alkylgruppen mit 1 bis 12 C-Atomen,
- x steht für eine ganze Zahl von 1 bis 100, bevorzugt von 20 bis 30,
- y steht für eine ganze Zahl von 1 bis 20, bevorzugt von 2 bis 10 und
- a und b stehen für ganze Zahlen von 0 bis 50, bevorzugt von 10 bis 30.

Verbindungen die unter die oben genannten Formeln fallen, werden in den folgenden Patentanmeldungen, auf die explizit Bezug genommen wird, offenbart: US-A-4,122,029; US-A-4,265,878; US-A-4,421,769 und GB-A-2,066,659.

Besonders bevorzugte Dimethiconcopolyole im Sinne der Erfindung sind beispielsweise die kommerziell unter dem Handelsnamen SILWET (Union Carbide Corporation) und DOW CORNING (Dow) vertriebenen Produkte.

Erfindungsgemäß besonders bevorzugte Dimethiconcopolyole sind Dow Corning 190 und Dow Corning 193 (Dow).

Zusätzlich können die erfindungsgemäßen Mittel mindestens ein Proteinhydrolysat enthalten. Die Proteinhydrolysate sind Produktgemische, die durch sauer, basisch oder enzymatisch katalysierten Abbau von Proteinen (Eiweißen) erhalten werden.

Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Keratin DEC^{®} (Vincience), Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess 8 Co), Lexein^{®} (Inolex) und Kerasol^{®} (Croda) vertrieben.

Erfindungsgemäß bevorzugt ist die Verwendung von Proteinhydrolysaten pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex) und Crotein^{®} (Croda) erhältlich.

Wenngleich der Einsatz der Proteinhydrolysate als solche bevorzugt ist, können an deren Stelle gegebenenfalls auch anderweitig erhaltene Aminosäuregemische oder einzelne Aminosäuren wie beispielsweise Arginin, Lysin, Histidin oder Pyrroglutaminsäure eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Gluadin^{®} (Cognis), Lexein^{®} (Inolex), Crolastin^{®} (Croda) oder Crotein^{®} (Croda) vertrieben.

Bevorzugt sind die Proteinhydrolysate in einer Menge von 0,05 bis 5 Gew.-%, besonders bevorzugt von 0,5 bis 2,0 Gew.-%, jeweils bezogen auf das Gewicht des anwendungsbereiten Mittels, enthalten.

In einer weiteren Ausführungsform enthalten die erfindungsgemäßen Mittel zusätzlich mindestens ein kationisches und/oder mindestens ein amphoteres Polymer.

Unter kationischen Polymeren sind Polymere zu verstehen, welche in der Haupt- und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen. Homopolymere der allgemeinen Formel (P1), 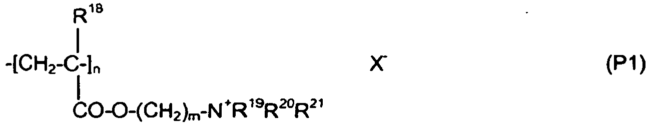 in der R¹⁸ = -H oder -CH₃ ist, R¹⁹, R²⁰ und R²¹ unabhängig voneinander ausgewählt sind aus C₁₋₄-Alkyl-, -Alkenyl- oder -Hydroxyalkylgruppen, m = 1, 2, 3 oder 4, n eine natürliche Zahl und X ein physiologisch verträgliches organisches oder anorganisches Anion ist, sowie Copolymere, bestehend im wesentlichen aus den in Formel (III) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹⁸ steht für eine Methylgruppe
- R¹⁹, R²⁰ und R²¹ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (III) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- Polysiloxane mit quaternären Gruppen, wie beispielsweise die im Handel erhältlichen Produkte Q2-7224 (Hersteller: Dow Coming; ein stabilisiertes Trimethylsilylamodimethicon), Dow Corning^{®} 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abit^{®}-Quat 3270 und 3272 (Hersteller: Th. Goldschmidt; diquaternäre Polydimethylsiloxane, Quatemium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden.
- quaternierter Polyvinylalkohol,
sowie die unter den Bezeichnungen
- Polyquaternium 2 (z.B. Mirapol® A-15 der Firma Rhodia),
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben.

Besonders gut geeignete Chitosane weisen einen Deacetylierungsgrad von wenigstens 80 % und ein Molekulargewicht von 5 · 10⁵ bis 5 · 10⁶ (g/mol) auf.

Zur Herstellung erfindungsgemäßer Zubereitungen muß das Chitosan in die Salzform überführt werden. Dies kann durch Auflösen in verdünnten wäßrigen Säuren erfolgen. Als Säuren sind sowohl Mineralsäuren wie z.B. Salzsäure, Schwefelsäure und Phosphorsäure als auch organische Säuren, z.B. niedermolekulare Carbonsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z.B. Essigsäure, Glycolsäure, Weinsäure, Apfelsäure, Citronensäure, Milchsäure, 2-Pyrrolidinon-5-carbonsäure, Benzoesäure oder Salicylsäure. Bevorzugt werden niedermolekulare Hydroxycarbonsäuren wie z.B. Glycolsäure oder Milchsäure verwendet.

Unter dem Begriff amphotere Polymere werden solche Polymere verstanden,
- die im Molekül sowohl freie Aminogruppen als auch freie -COOH- oder SO₃H-Gruppen enthalten und zur Ausbildung innerer Salze befähigt sind,
- zwitterionische Polymere, die im Molekül quartäre Ammoniumgruppen und -COO⁻-Gruppen oder -SO₃⁻-Gruppen enthalten, sowie
- Polymere, die -COOH-Gruppen oder SO₃H-Gruppen und quartäre Ammoniumgruppen enthalten.
Die in der Aufzählung genannten Polymere mit quartären Ammoniumgruppen werden erfindungsgemäß bevorzugt als amphotere Polymere eingesetzt.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Erfindungsgemäß bevorzugte amphotere und/oder kationische Polymere sind solche Polymerisate, in denen sich eine kationische Gruppe ableitet von mindestens einem der folgenden Monomere:
- Monomeren mit quartären Ammoniumgruppen der allgemeinen Formel (M1),

   R¹-CH=CR²-CO-Z-(CₙH₂ₙ)-N⁽⁺⁾R³R⁴R⁵ A⁽⁻⁾ (M1)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist,
- Monomeren mit quartären Ammoniumgruppen der allgemeinen Formel (M2), 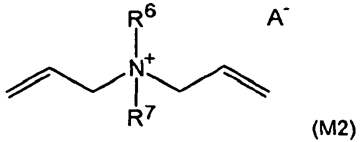 worin R⁶ und R⁷ unabhängig voneinander stehen für eine (C₁ bis C₄)-Alkylgruppe, insbesondere für eine Methylgruppe und
   A⁻ das Anion einer organischen oder anorganischen Säure ist.

Wenn sich eine kationische Gruppe der amphoteren bzw. kationischen Polymerisate vom Monomer der Formel (M1) ableitet, stehen die Reste R³, R⁴ und R⁵ in Formel (M1) bevorzugt für Methylgruppen, Z ist bevorzugt eine NH-Gruppe und A⁽⁻⁾ bedeutet bevorzugt ein Halogenid-, Methoxysulfat- oder Ethoxysulfat-Ion. Besonders bevorzugt ist es in diesem Falle Acryl-amidopropyl-trimethyl-ammoniumchlorid als Monomer (M1) zu verwenden.

In Formel (M2) steht A⁻ bevorzugt für ein Halogenidion, insbesondere für Chlorid oder Bromid.

Bevorzugte erfindungsgemäße amphotere Polymere sind Polymere, deren anionische Gruppe sich von mindestens einem Monomeren der Formel (M3) ableitet
- monomeren Carbonsäuren der allgemeinen Formel (M3) bzw. deren Salze mit einer organischen oder anorganischen Säure,

   R⁸-CH=CR⁹-COOH (M3)

   in denen R³ und R⁹ unabhängig voneinander Wasserstoff oder Methylgruppen sind.

Als Monomeres (M3) wird für die erfindungsgemäß bevorzugten amphoteren Polymerisate Acrylsäure verwendet.

Besonders bevorzugte amphotere Polymere sind Copolymere, aus mindestens einem Monomer (M1) bzw. (M2) mit dem Momomer (M3), insbesondere Copolymere aus den Monomeren (M2) und (M3). Erfindungsgemäß ganz besonders bevorzugt verwendete amphotere Polymere sind Copolymerisate aus Diallyl-dimethylammoniumchlorid und Acrylsäure. Diese Copolymerisate werden unter der INCI-Bezeichnung Polyquatemium-22 unter anderem mit dem Handelsnamen Merquat^{®} 280 (Nalco) vertrieben.

Darüber hinaus können die erfindungsgemäßen amphoteren Polymere neben einem Monomer (M1) oder M(2) und einem Monomer (M3) zusätzlich ein Monomer (M4)
- monomere Carbonsäureamide der allgemeinen Formel (M4), 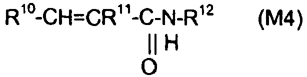 in denen R¹⁰ und R¹¹ unabhängig voneinander Wasserstoff oder Methylgruppen sind und R¹² für ein Wasserstoffatom oder eine (C₁- bis C₈)-Alkylgruppe steht, enthalten.

Erfindungsgemäß ganz besonders bevorzugt verwendete amphotere Polymere auf Basis eines Comonomers (M4) sind Terpolymere aus Diallyldimethylammoniumchlorid, Acrylamid und Acrylsäure. Diese Copolymerisate werden unter der INCI-Bezeichnung Polyquaternium-39 unter anderem mit dem Handelsnamen Merquat^{®} Plus 3330 (Nalco) vertrieben.

Besonders bevorzugt eingesetzte amphotere Polymere sind solche Polymerisate, die sich im wesentlichen zusammensetzen aus
(i) Monomeren mit quartären Ammoniumgruppen der allgemeinen Formel (M1),

   R¹-CH=CR²-CO-Z-(CₙH₂ₙ)-N⁽⁺⁾R²R³R⁴ A⁽⁻⁾ (M1)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist
   und
(ii) monomeren Carbonsäuren der allgemeinen Formel (M3),

   R⁸-CH=CR⁹-COOH (M3)

   in denen R⁰ und R⁹ unabhängig voneinander Wasserstoff oder Methylgruppen sind.

Bezüglich der Einzelheiten der Herstellung dieser besonders bevorzugten Polymerisate wird ausdrücklich auf den Inhalt der deutschen Offenlegungsschrift 39 29 973 Bezug genommen. Ganz besonders bevorzugt sind solche Polymerisate, bei denen Monomere des Typs (i) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethylammoniumchlorid ist ein besonders bevorzugtes Monomeres (i). Als Monomeres (ii) für die genannten Polymerisate wird bevorzugt Acrylsäure verwendet.

Die amphoteren Polymere können generell sowohl direkt als auch in Salzform, die durch Neutralisation der Polymerisate, beispielsweise mit einem Alkalihydroxid, erhalten wird, erfindungsgemäß eingesetzt werden.

In einer weiteren bevorzugten Ausführungsform der Erfindung kann die Wirkung durch Zusatz von mindestens einem Fettstoff weiter optimiert werden. Unter Fettstoffen sind zu verstehen Fettsäuren, Fettalkohole, natürliche und synthetische Wachse, welche sowohl in fester Form als auch flüssig in wäßriger Dispersion vorliegen können, und natürliche und synthetische kosmetische Ölkomponenten zu verstehen.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. In einer bevorzugten Ausführungsform beträgt die Menge 0,5 - 10 Gew.%, wobei ganz besonders vorteilhaft Mengen von 1 - 5 Gew.% sind.

Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfndungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden. Die Fettalkohole werden in Mengen von 0,1 - 20 Gew.-%, bezogen auf die gesamte Zubereitung, bevorzugt in Mengen von 0,1 - 10 Gew.-% eingesetzt.

Als natürliche oder synthetische Wachse können erfindungsgemäß eingesetzt werden feste Paraffine oder Isoparaffine, Camaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Zu den natürlichen und synthetischen kosmetischen Ölkörpern, welche die Wirkung des erfindungsgemäßen Wirkstoffes steigern können, sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyln-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®}OE) können bevorzugt sein.
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methytestern auf Basis von Fetten und Ölen oder Aldehyden aus der Roelen'schen Oxosynthese sowie als Monomerfraktion bei der Dimerisierung von ungesättigten Fettalkoholen anfallen. Efindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexylradipat, Di-(2-ethylhexy)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Mono,- Di- und Trifettsäureester von gesättigten und/oder ungesättigten linearen und/oder verzweigten Fettsäuren mit Glycerin, wie beispielsweise Monomuls^{®} 90-O18, Monomuls^{®} 90-L12 oder Cutina^{®} MD.

Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Die Gesamtmenge an Öl- und Fettkomponenten in den erfindungsgemäßen Mitteln beträgt üblicherweise 6 - 45 Gew.-%, bezogen auf das gesamte Mittel. Mengen von 10- 35 Gew.-% sind erfindungsgemäß bevorzugt.

Weiterhin hat sich gezeigt, daß die Wirkung gesteigert werden kann, wenn die erfindungsgemäßen Mittel zusätzlich mindestens einen Hydroxycarbonsäureester enthalten. Bevorzugte Hydroxycarbonsäureester sind Vollester der Glycolsäure, Milchsäure, Äpfelsäure, Weinsäure oder Citronensäure. Weitere grundsätzlich geeigneten Hydroxycarbonsäureester sind Ester der ß-Hydroxypropionsäure, der Tartronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 - 22 C-Atomen, also z.B. Fettalkohole oder synthetische Fettalkohole. Dabei sind die Ester von C₁₂-C₁₅-Fettalkoholen besonders bevorzugt. Ester dieses Typs sind im Handel erhältlich, z.B. unter dem Warenzeichen Cosmacol^{®} der EniChem, Augusta Industriale. Die Einsatzmenge der Hydroxycarbonsäureester beträgt dabei 0,1 - 15 Gew.% bezogen auf das Mittel, bevorzugt 0,1 - 10 Gew.% und ganz besonders bevorzugt 0,1 - 5 Gew.%.

Es ist efindungsgemäß bevorzugt, dass die Mittel zusätzlich mindestens ein Alkalisierungsmittel enthalten. Die erfindungsgemäß verwendbaren Alkalisierungsmittel werden bevorzugt ausgewählt aus der Gruppe, die gebildet wird, aus Ammoniak, basischen Aminosäuren, Alkalihydroxiden, Alkanolaminen; Alkalimetallmetasilikaten, Harnstoff, Morpholin, N-Methylglucamin, Imidazol, Alkaliphosphaten und Alkalihydrogenphosphaten. Als Alkalimetallionen dienen bevorzugt Lithium, Natrium, Kalium, insbesondere Natrium oder Kalium.

Die als erfindungsgemäßes Alkalisierungsmittel einsetzbaren basischen Aminosäuren werden bevorzugt ausgewählt aus der Gruppe, die gebildet wird aus L-Arginin, D-Arginin, D,L-Arginin, L-Histidin, D-Histidin, D,L-Histidin, L-Lysin, D-Lysin, D,L-Lysin, besonders bevorzugt L-Arginin, D-Arginin, D,L-Arginin als ein Alkalisierungsmittel im Sinne der Erfindung eingesetzt.

Die als erfindungsgemäßes Alkalisierungsmittel einsetzbaren Alkalihydroxide werden bevorzugt ausgewählt aus der Gruppe, die gebildet wird aus Natriumhydroxid und Kaliumhydroxid.

Die als erfindungsgemäßes Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-l-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol.

Besonders bevorzugt wird das Alkalisierungsmittel ausgewählt aus mindestens einer Verbindung aus der Gruppe, die gebildet wird aus 2-Aminoethanol, 2-Amino-2-methylpropan-1-ol, 2-Amino-2-methyl-propan-1,3-diol, Kaliumhydroxid, L-Arginin, D-Arginin, DL-Arginin, N-Methylglucamin, Morpholin, Imidazol und Hamstoff.

In einer weiteren Ausführungsform sollten die erfindungsgemäßen Mittel zusätzlich mindestens einen UV-Lichtschutzfilter enthalten. Unter UV-Lichtschutzfiltern sind organische Substanzen zu verstehen, die in der Lage sind, ultra-violette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substan-zen sind z.B. zu nennen:
- 3-Benzylidencampher und dessen Derivate, z.B. 3-(4-Methylbenzyliden)campher;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester, 2-Cyano-3-phenyl-zimtsäure-2-ethylhexylester (Octocrylene);
- Ester der Salicylsäure, vorzugsweise Salicylsäure-2-ethylhexylester, Salicylsäure-4-isopropylben-zylester, Salicylsäurehomomenthylester;
- Derivate des Benzophenons, vorzugsweise 2-Hydroxy-4-methoxybenzophenon, 2-Hydroxy-4-meth-oxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-carbo-2'-ethyl-1'-hexyloxy)-1,3,5-triazin und Octyltriazon.
- Propan-1,3-dione, wie z.B. 1-(4-tert.Butylphenyl)-3-(4'methoxyphenyl)propan-1,3-dion;

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sul-fonsäure und ihre Salze;
- Sulfonsäurederivate des 3-Benzylidencamphers, wie z.B. 4-(2-Oxo-3-bornylidenmethyl)benzolsul-fonsäure und 2-Methyl-5-(2-oxo-3-bornyliden)sulfonsäure und deren Salze.

Als typische UV-A-Filter kommen insbesondere Derivate des Benzoylmethans in Frage, wie beispiels-weise 1-(4'-tert.Butylphenyl)-3-(4'-methoxyphenyl)propan-1,3-dion oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C).

Weiterhin ist es bevorzugt, in die efindungsgemäßen Mittel zusätzlich mindestens ein Pflanzenextrakt einzuarbeiten.

Geeignete Pflanzenextrakte werden durch Extraktion mit organischen Lösemitteln (wie beispielsweise Ethanol, Isopropanol, Diethylether, Benzin, Benzol, Chloroform) oder durch Wasserdampfdestillation gewonnen. Bevorzugte Pflanzenextrakte sind beispielsweise Extrakte von Blüten (Lindenblüten, Kamillen, Lilie, Lavendel, Rosen, Jasmin, Neroli, Ylang-Ylang), Stengeln und Blättern (Cassis, Rosskastanie, Rooibos, Birke, Melisse, Klee, Weinblättem, Geranium, Patchouli, Petitgrain), Früchten (Anis, Cassis, Koriander, Kümmel, Wacholder), Fruchtschalen (Bergämotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian, Rosmarin), Nadeln und Zweigen (Fichte, Tanne, Kiefer, Latschen, Sandelholz), Harzen und Balsamen (Galbanum, Elemi, Benzoe, Myrrhe, Olibanum, Opoponax).

Besonders bevorzugt werden die Pflanzenextrakte ausgewählt aus mindestens einem Extrakt aus der Gruppe Hamamelis (Hamamelis virginiana L.), Weinblättern (Vitis vinifera L.), Rosen (Rosa gallica L.), Sandelholz (Pterocarpus Santalinus), Rooibos (Aspalathus linearis), Rosskastanie (Aesculus Hippocastanum L.), Klee (insbesondere Rotklee, Trifolium pratense), Zimt (Cinnamomum zeylanicum nees) und Cassis (insbesondere aus Cassis Blättern, Ribes nigrum L.).

Solche bevorzugt verwendeten Extrakte werden unter den Bezeichnungen Herbasol^{®} von der Firma Cosmetochem oder Extrapon^{®} von der Firma Symrise vermarktet.

Die Pflanzenextrakte sind in dem erfindungsgemäßen Mittel bevorzugt in einer Menge von 0,05 bis 5 Gew.-%, insbesondere 0,1 bis 2 Gew.-%, jeweils bezogen auf das Gewicht des gesamten Mittels, enthalten.

Ferner können die kosmetischen Mittel weitere Wirk-, Hitfs- und Zusatzstoffe enthalten, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- anionische Polymere wie beispielsweise Polyacrylsäuren, vernetzte Polyacrylsäuren, Vinylacetat/Crotonsäure-Copolymere, VinylpyrrolidonNinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Strukturanten wie Maleinsäure und Milchsäure,
- haarkonditionierende Verbindungen wie Phospholipide, beispielsweise Sojalecithin, Ei-Lecitin und Kephaline,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessemde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,
- Cholesterin,
- Konsistenzgeber wie Zuckerester, Polyolester oder Polyolalkylether,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wasserstoffperoxyd und andere Oxidationsmittel,
- Treibmittel wie Propan-Butan-Gemische, N₂O, Dimethylether, CO₂ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein zweiter Erfindungsgegenstand ist ein Verfahren zur Haarfarbveränderung, in dem ein kosmetisches Mittel, enthaltend in einem kosmetischen Träger mindestens eine farbverändemde Komponente und eine Kombination aus
(i) mindestens einer Verbindung gemäß Formel (I), 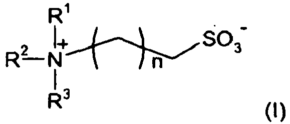 worin
   - R¹, R² und R³: stehen unabhängig voneinander für ein Wasserstoffatom, -CH₃, -CH₂CH₃, -CH(CH₃)₂, -CH₂CH₂CH₃, -CH(CH₃)CH₂CH₃, -CH₂CH(CH₃)₂, -C(CH₃)₃,
   - n: steht für 1 oder 2,
   und
(ii) mindestens einer organischen Disulfidverbindung mit einem Molekulargewicht von kleiner 700 g/mol,
auf das Haar aufgetragen wird und nach einer Einwirkungszeit vom Haar gespült wird.

Die Einwirkungszeit des Mittels beträgt bevorzugt 1 bis 100 Minuten, besonders bevorzugt 5 bis 50 Minuten.

Es ist wiederum erfindungsgemäß bevorzugt das erfindungsgemäße Verfahren im Rahmen einer oxidativen Haarfärbung oder der oxidativen Haarbleiche anzuwenden.

In einer bevorzugten Ausführungsform des erfindungsgemäßen Verfahrens als oxidatives Haarfärbeverfahren, wird bevorzugt die Kombination aus Verbindungen der Formel (I) und der erfindungsgemäßen organischen Disulfidverbindungen als Bestandteil eines kosmetischen Mittels enthaltend in einem kosmetischen Träger zusätzlich mindestens ein Oxidationsmittel und mindestens ein Oxidationsfarbstoffvorprodukt in einem Schritt angewendet.

Die oxidativen Haarfärbemittel dieser Ausführungsform sind bevorzugt Zweikomponenten Mittel. Die erste Komponente enthält in einem kosmetischen Träger die Kombination aus mindestens einer Verbindung der Formel (I) und mindestens einer organischen Disulfidverbindung mit einem Molekulargewicht kleiner 700 g/mol und mindestens ein Oxidationsfarbstoffvorprodukt. Die zweite Komponente enthält in einem kosmetischen Träger mindestens ein Oxidationsmittel. Diese Komponenten werden bevorzugt getrennt voneinander in jeweils einem Kompartiment konfektioniert und gemeinsam in einer Verpackungseinheit (Kit) bereitgestellt. Kurz vor der Anwendung werden beide Komponenten miteinander vermischt.

In der Ausführungsform des erfindungsgemäßen Verfahrens als oxidatives Haarbleichverfahren, werden bevorzugt mindestens eine Verbindung der Formel (I) und mindestens eine organische Disulfidverbindung mit einem Molekulargewicht kleiner 700 g/mol in einem kosmetischen Mittel enthaltend in einem kosmetischen Träger zusätzlich mindestens ein Oxidationsmittel und mindestens einen Bleichverstärker in einem Schritt angewendet.

Die Haarbleichmittel dieser Ausführungsform sind bevorzugt Zwei- oder Dreikomponenten-Mittel. Die erste Komponente enthält in einem kosmetischen Träger die Verbindungen der Formel (I) und die erfindungsgemäßen organischen Disulfidverbindungen und gegebenenfalls mindestens einen Bleichverstärker. Der Bleichverstärker kann auch getrennt von den Verbindungen der Formel (I) und den erfindungsgemäßen organischen Disulfidverbindungen konfektioniert sein, beispielsweise in Pulverform, als wasserfreie Paste oder wasserfreies Öl. Dadurch wird ein Dreikomponenten-Mittel erhalten. Die letzte Komponente enthält in einem kosmetischen Träger mindestens ein Oxidationsmittel. Alle Komponenten werden bevorzugt getrennt voneinander in jeweils einem Kompartiment konfektioniert und gemeinsam in einer Verpackungseinheit (Kit) bereitgestellt. Kurz vor der Anwendung werden alle Komponenten miteinander gemischt.

Die zuvor beschriebenen Mehrkomponentenmittel zur Durchführung des erfindungsgemäßen Verfahrens können in einer Verpackungseinheit bereitgestellt werden. Die Verpackungseinheit kann mindestens entweder einen Kontainer umfassen, der das Mittel des zweiten Erfindungsgegenstandes enthält, oder kann mindestens zwei Kontainer umfassen, wobei ein erster Kontainer ein oxidatives kosmetisches Mittel, enthaltend in einem kosmetischen Träger mindestens ein Oxidationsmittel, enthält, ein zweiter Kontainer ein kosmetisches Mittel, enthaltend in einem kosmetischen Träger mindestens eine Verbldnung der Formel (I) in Kombination mit mindestens einer organischen Disulfidverbindung mit einem Molekulargewicht kleiner 700 g/mol, enthält und gegebenenfalls ein dritter Kontainer, der mindestens einen Bleichverstärker, eingebettet in einem kosmetischen Träger, enthält. Alle Kontainer können auch Kammern eines Mehrkammerbehältnisses sein.

Für den zweiten Erfindungsgegenstand gilt mutatis mutandis das für den ersten Erfindungsgegenstand Gesagte.

Der dritte Erfindungsgegenstand ist die nicht-therapeutische Verwendung eines Mittels des ersten Erfindungsgegenstandes zur kopfhautschonenden Haarfarbveränderung. Bevorzugt eignet sich das Mittel des ersten Erfindungsgegenstandes zur Linderung sensorischer Missempfindungen auf der Kopfhaut, wie insbesondere Stechen oder Juckreiz.

Für den dritten Erfindungsgegenstand gilt mutatis mutandis das für den ersten und zweiten Erfindungsgegenstand Gesagte.

Die folgenden Beispiele sollen den Gegenstand der vorliegenden Erfindung erläutern ohne ihn jedoch zu beschränken.

### Beispiele

Die folgenden Rezepturen wurden unter Anwendung bekannter Herstellungsverfahren bereitgestellt. Alle Mengenangaben sind, falls nicht anders gekennzeichnet, Gewichtsprozent bezogen auf das Gewicht der jeweiligen Rezeptur.

Es wurden nachstehende Handelsprodukte als Rohstoffe verwendet:

| | |
|---|---|
| Hydrenol^{®} D | C₁₆₋C₁₈-Fettalkohol (INCI-Bezeichnung: Cetearyl alcohol) (Cognis Deutschland) |
| Lorol^{®} techn. | C₁₂-C₁₈-Fettalkohol (INCI-Bezeichnung: Coconut alcohol) (Cognis Deutschland) |
| Stenol^{®} 16/18 | C₁₆-₁₈-Fettalkohol (INCI-Bezeichnung: Cetearyl Alcohol) (Cognis) |
| Lorol^{®} 16 | INCI-Bezeichnung: Cetyl Alcohol (Cognis) |
| Eumulgin^{®} B1 | Cetylstearylakohol mit 12 EO-Einheiten (INCI-Bezeichnung: Ceteareth-12) (Cognis Deutschland) |
| Eumulgin^{®} B2 | Cetylstearylalkohol mit ca. 20 EO-Einheiten (INCI-Bezeichnung: Ceteareth-20) (Cognis Deutschland) |
| Edenor^{®} C14 | Myristinsäure (INCI-Bezeichnung: Myristic Acid) (Cognis) |
| Turpinal^{®} SL | 1-Hydroxyethan-1,1-diphosphonsäure (INCI-Bezeichnung: Etidronic Acid, Aqua (Water)) (Solutia) |
| Inutec^{®} H25 | Oligofructosehaltiges Inulin Hydrolysat, auch als Raftilose bezeichnet (93 Gew.-% Oligofructose, INCI-Bezeichnung: Inulin) (Orafti) |
| Disponil^{®} FES 77 IS | Fettalkoholethersulfat (ca. 31-33% Aktivsubstanzgehalt in Wasser; INCI-Bezeichnung: Sodium Coceth-30 Sulfate) (Cognis) |
| Akypo^{®} Soft 45 NV | 2-(C₁₂₋₁₄-Fettalkoholethoxylat (4.5 EO))-essigsäure Natriumsalz; 21% Aktivsubstanz; INCl-Bezeichnung: Sodium Laureth-5 Carboxylate (KAO) |
| Protelan^{®} MST 35 | Gemisch aus Natrium N-Myristoylsarcosinate und Natrium N-Methyl-N-cocoyltaurate (40 Gew.-% Aktivsubstanz, Zschimmer & Schwartz) |
| Aculyn^{®} 33 | 30 Gew.-% Aktivsubstanz in Wasser (INCI-Bezeichnung: Acrylates Copolymer) (Rohm & Haas) |
| Polymer^{®} W 37194 | ca. 20Gew.-% Aktivsubstanzgehalt in Wasser; INCI-Bezeichnung: Acrylamidopropyttrimonium Chloride/Acrylates Copolymer (Stockhausen) |

### 1.0 offener Epikutantest

Der offene Epikutantest nach COLIPA (Test Guidelines for the Assessment of Human Skin Tolerance, 1997) ist geeignet, Substanzen und Fertigprodukte auf Hautverträglichkeit zu prüfen. Es wurden 20 freiwillige weibliche und männliche hautgesunde Probanden ohne Altersbegrenzung in die Prüfung einbezogen. Die Cremes F1, F2, F3 und F4 der Tabelle 1 wurden jeweils im Gewichtsverhältnis 1 zu 1 mit dem Entwickler E der Tabelle 1 gemischt. Die zuvor beschriebene Mischung aus der Creme F4 mit dem Entwickler E ergibt ein erfindungsgemäßes Mittel. Alle anderen Mittel sind nicht erfindungsgemäß und dienten zum Vergleich.

**Tabelle 1: Testrezepturen**

| | F1 | F2 | F3 | F4 | E |
|---|---|---|---|---|---|
| Hydrenol D | 7,15 | 7,15 | 7,15 | 7,15 | - |
| Lorol techn. | 2,60 | 2,60 | 2,60 | 2,60 | 3,6 |
| Eumulgin B 1 | 0,65 | 0,65 | 0,65 | 0,65 | - |
| Eumulgin B 2 | 0,65 | 0,65 | 0,65 | 0,65 | 0,9 |
| Turpinal SL | 0,20 | 0,20 | 0,20 | 0,20 | 1,5 |
| Akypo Soft 45 NV | 10,0 | 10,0 | 10,0 | 10,0 | - |
| Protelan MST 35 | 6,0 | 6,0 | 6,0 | 6,0 | - |
| Polymer W 37194 | 3,75 | 3,75 | 3,75 | 3,75 | - |
| Inutec H25 | 1,00 | 1,00 | 1,00 | 1,00 | - |
| Monoethanolamin | 2,00 | 2,00 | 2,00 | 2,00 | - |
| Natriumchlorid | 2,00 | 2,00 | 2,00 | 2,00 | - |
| Natriumsulfit | 0,40 | 0,40 | 0,40 | 0,40 | - |
| Ascorbinsäure | 0,10 | 0,10 | 0,10 | 0,10 | - |
| Natriumsilikat 40/42 | 0,50 | 0,50 | 0,50 | 0,50 | - |
| Kaliumhydroxid 50% | 1,00 | 1,00 | 1,00 | 1,00 | - |
| Glycin | 1,00 | 1,00 | 1,00 | 1,00 | - |
| 2-Ammonioethansulfonat | - | - | 1,00 | 1,00 | - |
| α-Liponsäure | - | 0,20 | - | 0,20 | - |
| Aculyn 33A | - | - | - | - | 15,0 |
| Natronlauge 45 % | - | - | - | - | 0,73 |
| Disponil FES 77 | - | - | - | - | 2,25 |
| Dipicolinsäure | - | - | - | - | 0,10 |
| Dinatriumpyrophosphat | - | - | - | - | 0,03 |
| Wasserstoffperoxid 50 % | - | - | - | - | 12,0 |
| Wasser | ad. 100 | ad. 100 | ad. 100 | ad. 100 | ad. 100 |

Je Testperson wurden an randomisierten Positionen die fertigen drei Anwendungsmischungen mit einem Glasstab auf der Unterarminnenseite über einen Zeitraum von 30 Minuten alle 30 Sekunden appliziert. Nach Applikationsende wurden die Substanzen vorsichtig mit Wasser vom Unterarm abgewaschen und die Hautareale getrocknet. Die Testpersonen wurden anschließend auf eine Hautrötung an den Hautarealen überprüft. Ferner wurde abgefragt, ob die Testpersonen einen Juckreiz verspüren und falls ja an welchem Hautareal. Die Testergebnisse sind der Tabelle 2 zu entnehmen und zeigen die Anzahl der Probanden in Prozent, die mindestens eine der abgefragten Hautreaktionen aufwiesen.

**Tabelle 2: Testergebnisse**

| Mischung | Reaktionen der Probanden |
|---|---|
| F1 / E | 60% |
| F2 / E | 50 % |
| F3 / E | 35 % |
| F4 / E | 25 % |

Die erfindungsgemäße Mischung F4 / E mit der Synergie aus einer erfindungsgemäßen Aminosulfonsäure (2-Ammonioethansulfonat) und einer erfindungsgemäßen organischen Disulfidverbindung (α-Liponsäure) bewirkte erheblich weniger Missempfindungen auf der Haut der Probanden, als die restlichen Vergleichsmischungen.

## Patentansprüche

1. Kosmetisches Mittel, enthaltend in einem kosmetischen Träger mindestens eine haarfarbverändernde Komponente **dadurch gekennzeichnet, daß** es zusätzlich eine Kombination aus
(i) mindestens einer Verbindung gemäß Formel (I), 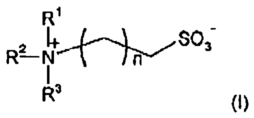 worin
R¹, R² und R³ stehen unabhängig voneinander für ein Wasserstoffatom, -CH₃, -CH₂CH₃, -CH(CH₃)₂, -CH₂CH₂CH₃, -CH(CH₃)CH₂CH₃, -CH₂CH(CH₃)₂, -C(CH₃)₃,
n steht für 1 oder 2,
und
(ii) mindestens einer organischen Disulfidverbindung mit einem Molekulargewicht von kleiner 700 g/mol enthält.

2. Mittel nach Anspruch 1. **dadurch gekennzeichnet, daß** die haarfarbverändernde Komponente ausgewählt wird
(a) aus mindestens einem Oxidationsfarbstoffvorprodukt vom Typ der Entwicklerkomponenten und gegebenenfalls zusätzlich mindestens einer Kupplerkomponente
und/oder
(b) aus Oxofarbstoffvorprodukten
und/oder
(c) aus mindestens einem direktziehenden Farbstoff
und/oder
(d) aus mindestens einer Vorstufe naturanaloger Farbstoffe
und/oder
(e) aus mindestens einem Oxidationsmittel und gegebenenfalls zusätzlich mindestens einem Bleichverstärker.

3. Mittel nach einem der Ansprüche 1 oder 2, **dadurch gekennzeichnet, daß** es die Verbindungen der-Formel (I) in einer Menge von 0.01 bis 7 Gew.-%, bezogen auf das Gewicht des anwendungsbereiten Mittels, enthält.

4. Mittel nach einem der Ansprüche 1 bis 3, **dadurch gekennzeichnet, daß** die Verbindung der Formel (I) ausgewählt wird, aus mindestens einer Verbindung der Gruppe, die gebildet wird aus 2-Ammonioethansulfonat (Taurin), 2-(N-Methylammonio)ethansulfonat, 2-(N,N-Dimethyl-ammonio)ethansulfonat, 2-(N,N,N-Trimethylammonio)ethansulfonat 3-Ammoniopropan-sulfonat (Homotaurin), 3-(N-Methylammonio)propansulfonat, 3-(N,N-Dimethylammonio)-propansultonat, 3-(N,N,N-Trimethylammanio)propansulfonat.

5. Mittel nach einem der Ansprüche 1 bis 4, **dadurch gekennzeichnet, daß** die organische Disulfidverbindung ausgewählt wird aus mindestens einer Verbindung uns der Gruppe, die gebildet wird aus Cystin und α-Liponsäure, sowie den Salzen der vorgenannten Verbindungen.

6. Mittel nach einem der Ansprüche 1 bis 6, **dadurch gekennzeichnet, daß** die organische Disulfidvervindung in einer Menge von 0,01 bis 3,0 Gew.-%, bezogen auf das Gewicht des anwendungsbereiten Mittels, enthalten ist.

7. Mittel nach einem der Ansprüche 1 bis 6, **dadurch gekennzeichnet, daß** es zusätzlich mindestens ein Tensid enthält.

8. Mittel nach einem der Anspruch 7, **dadurch gekennzeichnet, daß** es als Tensid mindestens eine Verbindung der Formel (X) und/oder der Formel (XI) enthält. 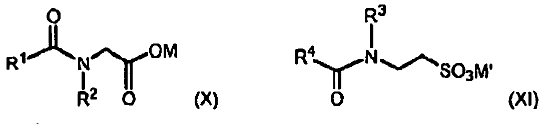 worin
R¹ eine (C₇ bis C₃₀)-Alkylgruppe, (C₇ bis C₃₀)-Alkenylgruppe oder eine (C₇ bis C₃₀). Hydroxyalkylgruppe bedeutet,
R² für eine (C₁ bis C₄)-Alkylgruppe oder eine (C₁ bis C₄)-Hydroxyalkylgruppe steht,
R³ für ein Wasserstoffatom, eine (C₁ bis C₄)-Alkylgruppe oder eine (C₁ bis C₄)-Hydroxyalkylgruppe steht,
R⁴ for eine (C₇ bis C₃₀)-Alkylgruppe, (C₇ bis C₃₀)-Alkenylgruppe oder eine (C₇ bis C₃₀)-Hydroxyalkylgruppe steht und
M und M' unabhängig vonainander ein Wasserstoffatom oder ein Äquivalent eines ein- oder mehrwertigen Kations bedeuten.

9. Verfahren zur Haarfarbveränderung, in dem ein kosmetisches Mittel nach einem der Ansprache 1 bis 8 auf das Haar aufgetragen wird und nach einer Einwirkungszeit vom Haar gespült wird.

10. Nicht-therapeutische Verwendung eines Mittels nach einem der Ansprüche 1 bis 8 zur kopfnautschonenden Haafarbveränderung.

## Claims

1. Cosmetic composition, comprising in a cosmetic carrier at least one hair colour-changing component, **characterised in that** the composition additionally comprises a combination of
(I) at least one compound according to Formula (I), 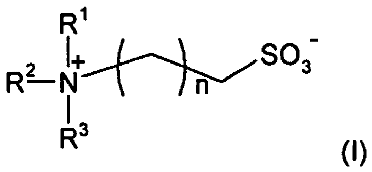 in which
R¹, R² and R³ stand independently of one another for a hydrogen atom,-CH₃, -CH₂CH₃, -CH(CH₃)₂, -CH₂CH₂CH₃,-CH(CH₃)CH₂CH₃, -CH₂CH(CH₃)₂, -C(CH₃)₃,
n stands for 1 or 2,
and
(II) at least one organic disulfide compound with a molecular weight of less than 700 g/mol.

2. Composition according to claim 1, **characterised in that** the hair colour-changing component is selected
(a) from at least one oxidation dye precursor of the developer component type and optionally additionally from at least one coupler component
and/or
(b) from oxo dye precursors
and/or
(c) from at least one substantive dye
and/or
(d) from at least one precursor of nature-analogous dyes
and/or
(e) from at least one oxidising agent and optionally additionally at least one bleach booster.

3. Composition according to one of claims 1 or 2, **characterised in that** it comprises the compounds of the Formula (I) in an amount of 0.01 to 7 wt %, based on the weight of the ready for use composition.

4. Composition according to one of claims 1 to 3, **characterised in that** the compound of the Formula (I) is selected from at least one compound of the group that is constituted from 2-ammonioethane sulfonate (taurine), 2-(*N*-methylammonio)ethane sulfonate, 2-(*N,N*-dimethylammonio)ethane sulfonate, 2-(*N*,*N*,*N*-trimethylammonio)ethane sulfonate, 3-ammoniopropane sulfonate (homotaurine), 3-(*N*-methylammonio)propane sulfonate, 3-(*N,N*-dimethylammonio)propane sulfonate, *3-(N,N,N-*trimethylammonio)propane sulfonate.

5. Composition according to one of claims 1 to 4, **characterised in that** the organic disulfide compound is selected from at least one compound of the group that is constituted from cystine and α-lipoic acid, as well as from the salts of the cited compounds.

6. Composition according to one of claims 1 to 5, **characterised in that** it comprises the organic disulfide compound in an amount of 0.01 to 3.0 wt %, based on the weight of the ready for use composition.

7. Composition according to one of claims 1 to 6, **characterised in that** it additionally comprising at least one surfactant.

8. Composition according to claim 7, **characterised in that** it comprises at least one compound of the Formula (X) and/or of the Formula (XI) as the surfactant, 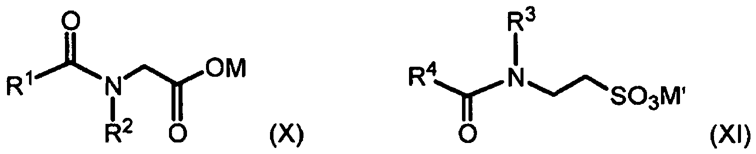 in which
R¹ means a (C₇ to C₃₀) alkyl group, (C₇ to C₃₀) alkenyl group or a (C₇ to C₃₀) hydroxyalkyl group,
R² stands for a (C₁ to C₄) alkyl group or a (C₁ to C₄) hydroxyalkyl group,.
R³ stands for a hydrogen atom, a (C₁ to C₄) alkyl group or a (C₁ to C₄) hydroxyalkyl group,
R⁴ stands for a (C₇ to C₃₀) alkyl group, (C₇ to C₃₀) alkenyl group or a (C₇ to C₃₀) hydroxyalkyl group and
M and M' independently of each other mean a hydrogen atom or an equivalent of a mono or multivalent cation.

9. Method for changing hair colour, in which method a cosmetic composition according to one of claims 1 to 8 is deposited on the hair and after a contact time is rinsed out of the hair.

10. Non-therapeutic use of a composition according to one of claims 1 to 8 for changing hair colour in a manner that is gentle to the scalp.

## Revendications

1. Agent cosmétique, contenant, dans un support cosmétique, au moins un composant modifiant la couleur des cheveux **caractérisé en ce qu'**il contient en plus une combinaison, constituée par
(i) au moins un composé selon la formule (I) 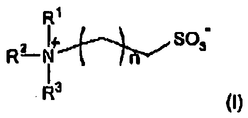 où
R¹, R² et R³ représentent, indépendamment les uns des autres, un atome d'hydrogène, -CH₃, -CH₂CH₃, -CH(CH₃)₂, -CH₂CH₂CH₃, -CH(CH₃)CH₂CH₃, -CH₂CH(CH₃)₂, -C(CH₃)₃,
n représente 1 ou 2,
et
(ii) au moins un composé disulfure organique présentant un poids moléculaire inférieur à 700 g/mole.

2. Agent selon la revendication 1, **caractérisé en ce que** le composant modifiant la couleur des cheveux est choisi parmi
(a) au moins un précurseur de colorant par oxydation du type des composants développeurs et le cas échéant en plus au moins un composant de couplage et/ou
(b) les précurseurs de colorants de type oxo et/ou
(c) au moins un colorant montant directement sur la fibre et/ou
(d) au moins un précurseur de colorants analogues aux colorants naturels et/ou
(e) au moins un oxydant et le cas échéant en plus au moins un agent de renforcement de la décoloration.

3. Agent selon l'une quelconque des revendications 1 à 2, **caractérisé en ce qu'**il contient les composés de formule (I) en une quantité de 0,01 à 7 % en poids, par rapport au poids de l'agent prêt à l'emploi.

4. Agent selon l'une quelconque des revendications 1 à 3, **caractérisé en ce que** le composé de formule (I) est choisi parmi au moins un composé du groupe formé par le 2-ammonioéthanesulfonate (taurine), le 2-(N-méthylammonio)éthanesulfonate, le 2-(N,N-diméthylammonio)éthanesulfonate, le 2-(N,N,N-triméthylammonio)éthanesulfonate, le 3-ammoniopropanesulfonate (homotaurine), le 3-(N-méthylammonio)propanesulfonate, le 3-(N,N-diméthylammonio)propanesulfonate, le 3-(N,N,N-triméthylammonio)propanesulfonate.

5. Agent selon l'une quelconque des revendications 1 à 4, **caractérisé en ce que** le composé disulfure organique est choisi parmi au moins un composé du groupe formé par la cystine et l'acide α-liponique, ainsi que les sels des composés susmentionnés.

6. Agent selon l'une quelconque des revendications 1 à 6, **caractérisé en ce que** le composé disulfure organique est contenu en une quantité de 0,01 à 3,0 % en poids, par rapport au poids de l'agent prêt à l'emploi.

7. Agent selon l'une quelconque des revendications 1 à 6, **caractérisé en ce qu'**il contient en plus au moins un agent tensioactif.

8. Agent selon la revendication 7, **caractérisé en ce qu'**il contient, comme agent tensioactif, au moins un composé de formule (X) et/ou de formule (XI), 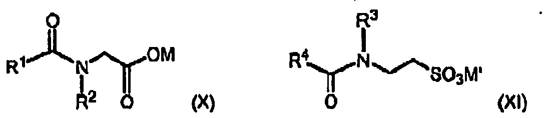 où
R¹ signifie un groupe (C₇ à C₃₀)-alkyle, un groupe (C₇ à C₃₀)-alcényle ou un groupe (C₇ à C₃₀)-hydroxyalkyle,
R² représente un groupe (C₁ à C₄)-alkyle ou un groupe (C₁ à C₄)-hydroxyalkyle,
R³ représente un atome d'hydrogène, un groupe (C₁ à C₄)-alkyle ou un groupe (C₁ à C₄)-hydroxyalkyle,
R⁴ représente un groupe (C₇ à C₃₀)-alkyle, un groupe (C₇ à C₃₀)-alcényle ou un groupe (C₇ à C₃₀)-hydroxyalkyle et
M et M' signifient, indépendamment l'un de l'autre, un atome d'hydrogène ou un équivalent d'un cation monovalent ou polyvalent.

9. Procédé de modification de la couleur des cheveux, dans lequel un agent cosmétique selon l'une quelconque des revendications 1 à 8 est appliqué sur les cheveux et éliminé par rinçage des cheveux après un temps d'action.

10. Utilisation non thérapeutique d'un agent selon l'une quelconque des revendications 1 à 8 pour la modification de la couleur des cheveux, ménageant le cuir chevelu.
